# EUROPEAN PATENT APPLICATION

(11) **EP 2 543 659 A1**
(43) Date of publication of application: **09.01.2013**
(21) Application number: 11719585.9
(22) Date of filing: 04.03.2011
(51) Int. Cl.: C07C 237/10, A61K 48/00, C12N 15/63, A61P 25/00, A61P 35/00

(54) **DENDRIMERS AS NON-VIRAL VEHICLES FOR GENE THERAPY**

(30) Priority: 05.03.2010 ES 201030322
(71) Applicant: Universidad De Castilla La Mancha, 02071 Albacete (ES)
(72) Inventor: CEÑA CALLEJO, Valentín, E-02071 Albacete (ES); SÁNCHEZ VERDU, Mª Del Prado, E-02071 Albacete (ES); MERINO GUIJARRO, Sonia, E-02071 Albacete (ES); GARCÍA MARTÍNEZ, Joaquín Calixto, E-02071 Albacete (ES); RODRÍGUEZ LÓPEZ, Julián, E-02071 Albacete (ES); VÁZQUEZ FERNÁNDEZ-PACHECO, Ester, E-02071 Albacete (ES); HERRERO CHAMORRO, María Antonia, E-02071 Albacete (ES); CAMPO RODRIGO, Ana, E-02071 Albacete (ES); RIVILLA DE LA CRUZ, Iván, E-02071 Albacete (ES); PÉREZ MARTÍNEZ, Francisco Carlos, E-02071 Albacete (ES); GUERRA NAVARRO, Francisco Javier, E-02071 Albacete (ES)
(74) Representative: Pons Ariño, Angel
(86) International application number: PCT/ES2011/070141
(87) International publication number: WO 2011/107648

(57) **Abstract**

The present invention relates to novel compounds of general formula (I) and (II) for their use in gene therapy as non-viral vehicles and their use for the preparation of a medicament. It also discloses the process of synthesis of said compounds of general formula (I) and (II).

## Description

The present invention relates to novel compounds of general formula (I), and (II) for their use in gene therapy as non-viral vehicles and their use for the preparation of a medicament. It also discloses the process of synthesis of said compounds of general formula (I) and (II).

### PRIOR ART

The use of non-viral vectors in gene therapy is especially relevant, since the FDA has suspended, sine die, the clinical tests using virus (adenovirus, adeno-associated virus, etc.) due to the fact that they generate immune reactions that have caused the death of some patients who participated in said tests. Viral vectors have several drawbacks, such as, for example, lack of safety in their handling, toxicity, provoking an immune response that decreases their efficacy or lack of cellular specificity. Together with this, these systems are quickly eliminated from the circulation, limiting the transfection process to first step organs (lungs, liver and spleen).

It is also necessary to bear in mind that recombination processes may originate a replicant virus although the danger is remote. Nevertheless, the problems posed by virus as vectors in gene therapy are serious and the clinical trials of gene therapy, for example, in the United States have been recently interrupted by the FDA due to the death of several patients due to multi-organ failure. This type of serious problems have led to the search for and development of alternatives to the use of virus as gene material vectors.

Non-viral vectors have a series of advantages with respect to viral analogues: a) ease in the preparation (even at multigram scale) and modification, b) greater flexibility with respect to the size of the genetic material to transfect c) they are generally safe in vivo and d) they do not cause a specific immune response and, therefore, they can be repetitively administered.

Within the non-viral vectors, dendrimers represent one of these alternatives, since they have a nanometric size, a globular structure, low polydispersability and a high functional density on the surface with a small molecular volume.

### DESCRIPTION OF THE INVENTION

The present invention relates to novel compounds of general formula (I), and (II) for their use in gene therapy as non-viral vehicles and their use for the preparation of a medicament or transfection kits. It also discloses the process of synthesis of said compounds of general formula (I) and (II).

Therefore, a first aspect of the present invention relates to a compound of general formula (I): 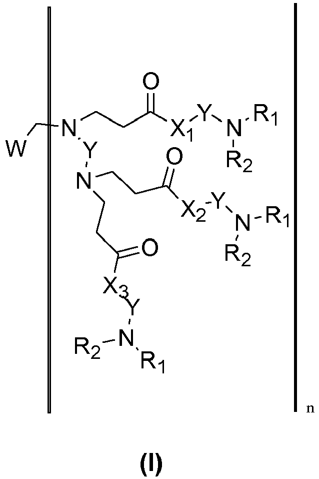 or a salt, prodrug or solvate thereof;
where:
W is selected from the group formed by any polymer or dendrimer derived from polyphenylenevinylene, polyphenyleneethylidene or hybrid of both, any conjugated organic compound that combines double bonds, triple bonds or a composition of both in its structure, any substitution with electron-donor or electro-attractor groups of the previous, any conjugated organic compound that alternates in its structure double bonds, triple bonds or a combination of both with aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous, any non-conjugated organic compound that contains aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous or any combination thereof.
X₁, X₂ and X₃ are the same or different and are selected from any heteroatom,
Y is selected from a saturated or unsaturated alkyl group (C₂-C₁₂) or cycloalkyl (C₃-C₈), cycloalkenyl, aryl or heteroaryl,
R₁ and R₂ are the same or different bound to a primary, secondary or tertiary amino group and are selected from hydrogen, saturated or unsaturated alkyl (C₁-C₁₂).
n represents the number of branches and is a whole number selected from between 1 and 10.

A preferred embodiment relates to compound of general formula (II): 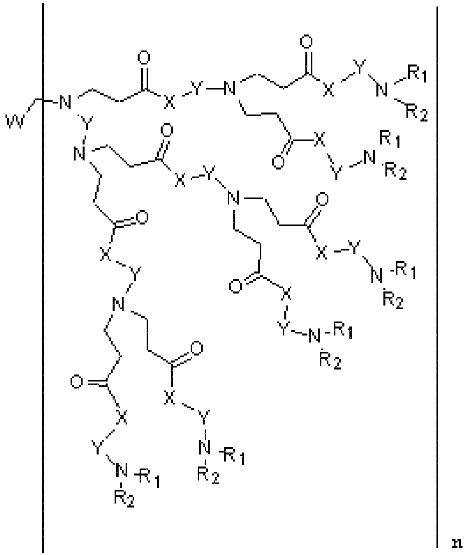 or a salt, prodrug or solvate thereof;
where:
W is selected from the group formed by any polymer or dendrimer derived from polyphenylenevinylene, polyphenyleneethylidene or hybrid of both, any conjugated organic compound that combines double bonds, triple bonds or a composition of both in its structure, any substitution with electron-donor or electro-attractor groups of the previous, any conjugated organic compound that alternates in its structure double bonds, triple bonds or a combination of both with aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous, any non-conjugated organic compound that contains aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous or any combination thereof.
X₁, X₂ and X₃ are the same or different and are selected from any heteroatom,
Y is selected from a saturated or unsaturated alkyl group (C₂-C₁₂) or cycloalkyl (C₃-C₈), cycloalkenyl, aryl or heteroaryl,
R₁ and R₂ are the same or different bound to a primary, secondary or tertiary amino group and are selected from hydrogen, saturated or unsaturated alkyl (C₁-C₁₂), cycloalkyl
n represents the number of branches and is a whole number selected from between 1 and 10.

In another preferred embodiment, the compounds of general formula (I) or (II) may be in the form of salt and electrostatically bound to trifluoroacetate, chloride anion, DNA, RNA, siRNA, miRNA, antagomir any drug, antibody, probe, (radioactive or not) for the diagnosis of diseases by imaging techniques (Nuclear Magnetic Resonance, Single photon emission computed tomography or Positron emission tomography) or any combination thereof

According to another preferred embodiment X₁, X₂ and X₃ are the same or different and are selected from nitrogen or oxygen.

In another preferred embodiment, both for the compound of general formula (I), and for those of general formula (II), X is selected without being limiting in nature from:

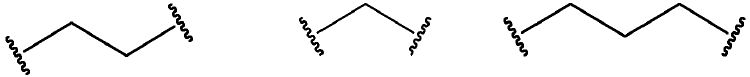

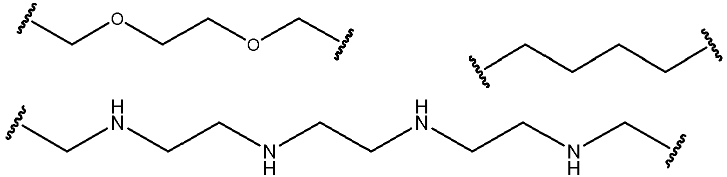

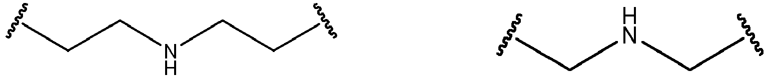

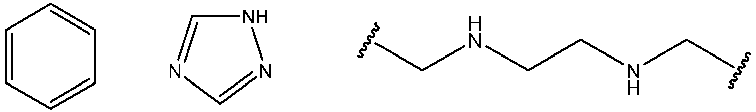

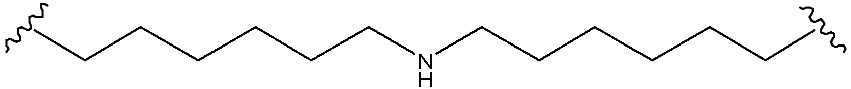

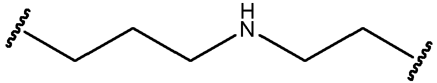

According to another preferred embodiment, the radicals R₁ to R₂ may be the same or different and are independently selected from H, any substituted or non-substituted amine, basic amino acids such as, for example, lysine or arginine, derivatives of cholesterol from cholesteryl chloroformiate, folic acid, lactic acid, dexamethasone, sugars such as, for example, and without being limiting in nature, lactose or mannose from their tosyl derivative, lysosomotropic agents such as, for example, chloroquine, nitrogenated heterocycles such as uridine, piperidine or piperazine, polyethylene glycol-derivative hydrophilic chains, any diacrylate, any basic amino acid and the following structures:

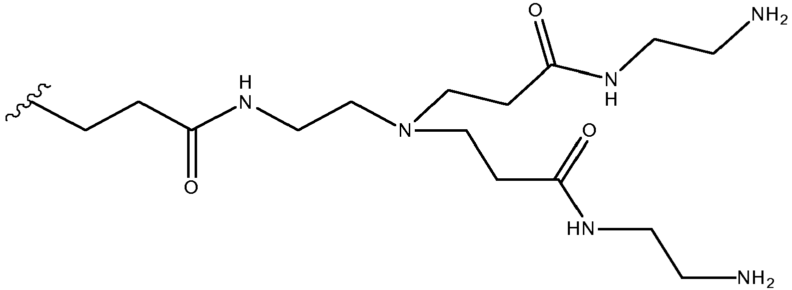

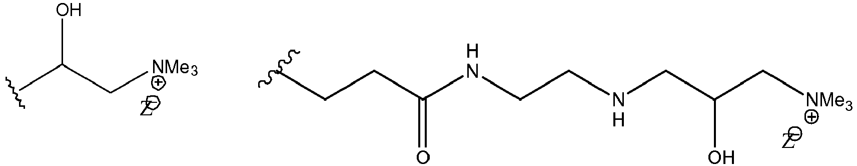

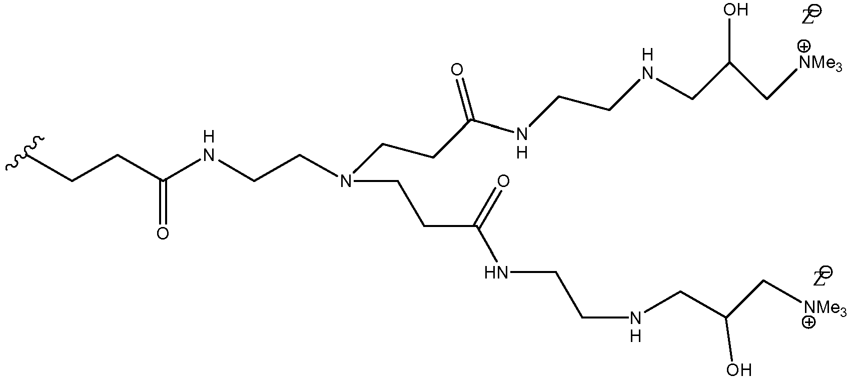

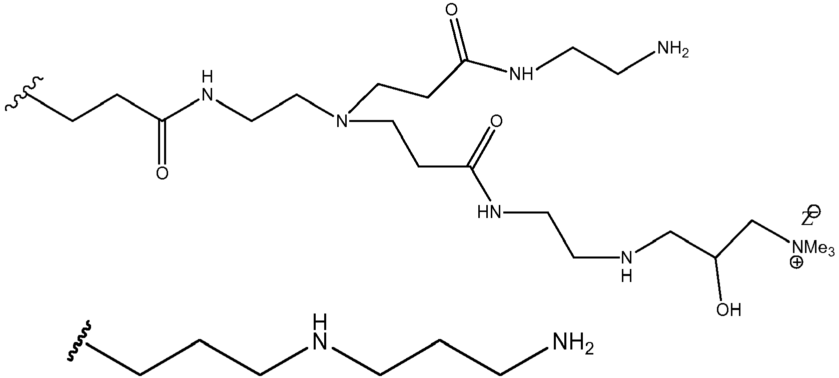

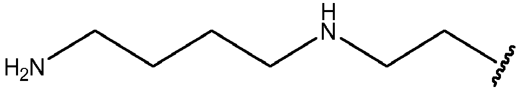

In a preferred embodiment, said compounds of general formula (I) or (II) are selected, without being limited to, the group comprising:

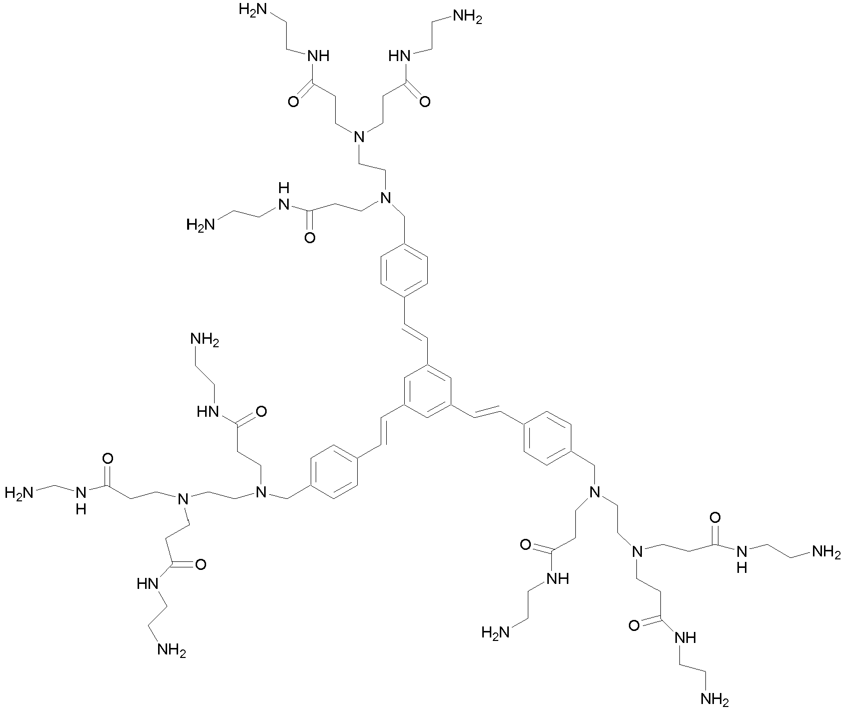

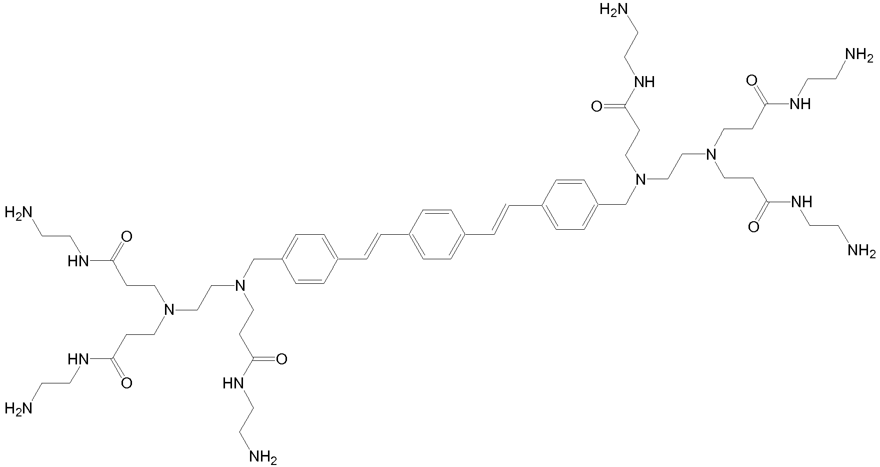

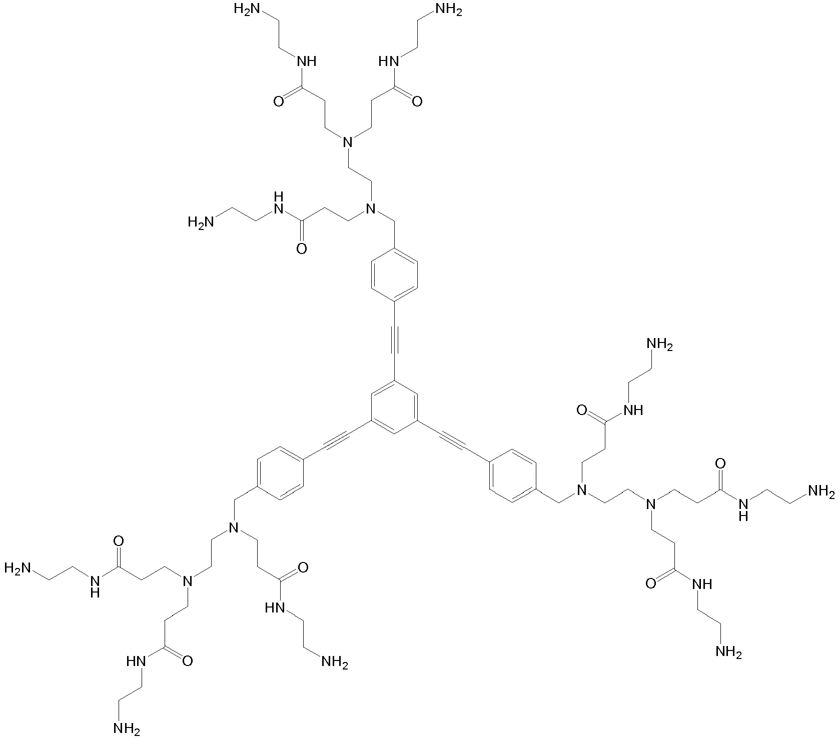

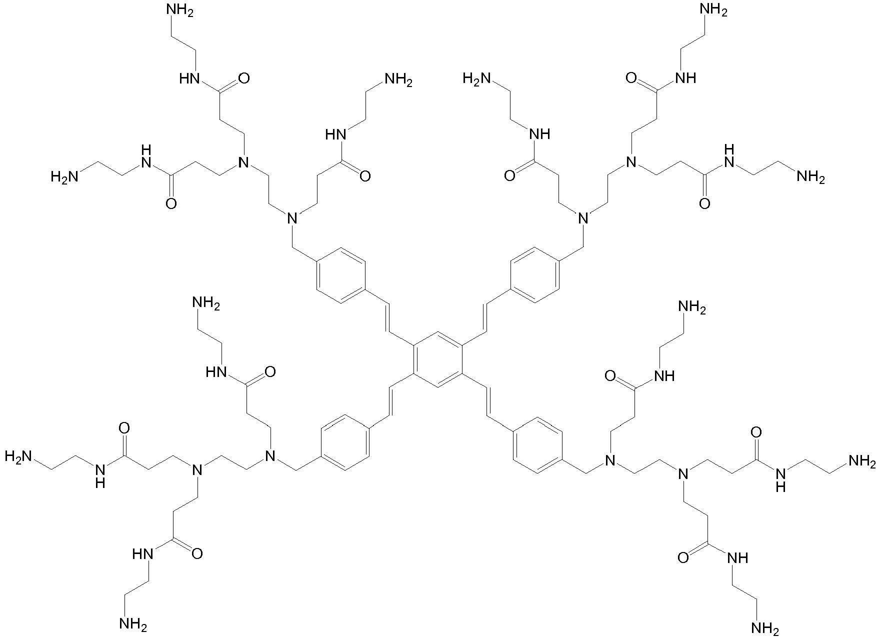

The term "alkyl" relates, in the present invention, to linear or branched, saturated or unsaturated aliphatic chains which have from 2 to 12 carbon atoms. For example, but without being limited to, methyl, ethyl, *n*-propyl, *i-*propyl, *n*-butyl, *tert*-butyl, *sec*-butyl, n-pentyl, n-hexyl, n-heptyl etc. Furthermore, the term alkyl also relates to linear or branched, saturated or unsaturated aliphatic chains from 2 to 12 carbon atoms, which may be substituted by functional groups such as, for example, hydroxyl, carboxyl, carbonyl, amine, amide or which may contain in its structure any heteroatom selected from nitrogen, oxygen and sulphur.

The term "cycloalkyl" relates to a stable monocyclic or bicyclic radical of 3 to 8 members that is saturated or partially saturated, and which only consists of carbon and hydrogen atoms. Such as, for example, but without being limited to, cyclobutane, cyclopentane, cyclohexane or cycloheptane. Furthermore, the term cycloalkyl also relates to a stable monocyclic or bicyclic radical of 3 to 8 members, that is saturated or partially saturated, and which only consists of carbon and hydrogen atoms, which may be substituted by functional groups such as, for example, hydroxyl, carboxyl, carbonyl, amine, amide or which may contain in its structure any heteroatom selected from nitrogen, oxygen and sulphur.

The term "pharmaceutically acceptable salts, solvates or prodrugs" relates to any pharmaceutically acceptable salt, ester, solvate, or any other compound which, when administered to a receptor is capable of providing (directly or indirectly) a compound as described in the present document. However, it shall be appreciated that the pharmaceutically unacceptable salts are also within the scope of the invention since they can be useful in the preparation of pharmaceutically acceptable salts. The preparation of salts, prodrugs and derivatives can be carried out by methods known in the state of the art.

For example, pharmaceutically acceptable salts of compounds provided in the present document are synthesized by conventional chemical methods from an original compound containing a basic or acid residue. Generally, said salts are prepared, for example, making the free base or acid forms of the compounds react with a stoichiometric quantity of the suitable base or acid in water or in an organic solvent or a mixture of both. Generally, non-aqueous media are preferred such as ether, ethyl acetate, ethanol, isopropanol or acetonitrile. Examples of acid addition salts include mineral acid addition salts such as, for example, hydrochloride, hydrobromide, hydriodide, sulfate, nitrate, phosphate and organic acid addition salts of such as, for example, acetate, maleate, fumarate, citrate, oxalate, succinate, tartrate, malate, mandelate, methanesulfonate and p-toluenesulfonate. Examples of base addition salts include inorganic salts such as, for example, sodium, potassium, calcium, ammonium, magnesium, aluminium and lithium salts, and salts of organic bases such as, for example, ethylenediamine, ethanolamine, N,N-dialkylenethanolamine, glucamine and salts of basic amino acids.

The particularly favourite derivatives or prodrugs are those that increase the bioavailability of the compounds of this invention when said compounds are administered to a patient (for example, making a compound administered by oral route be more easily absorbed by the blood), or which enhances the release of the original compound within a biological compartment (for example, the brain or lymphatic system) with relation to the original species.

Any compound that is a prodrug of a compound of formula (I) is within the scope of the invention. The term or "prodrug" is used in its broadest sense and covers those derivatives that are converted in vivo into the compounds of the invention. Said derivatives will be evident for those persons skilled in the art and include, depending on the functional groups present in the molecule and without limitation, the following derivatives of the compounds present: esters, amino acid esters, phosphate esters, metal salt sulfonate esters, carbamates and amino acid esters, sulfonate esters of metal salts, carbamates and amides.

The compounds of formula (I), (II) and (III) may be in crystalline form as free compounds or as solvates and it is aimed that both forms are within the scope of the present invention. The solvating methods are generally known within the state of the art. The suitable solvates are pharmaceutically acceptable solvates. In a particular embodiment, the solvate is a hydrate.

A second fundamental aspect of the present invention relates to a process for the preparation of a compound of general formula (I) comprising the following stages: 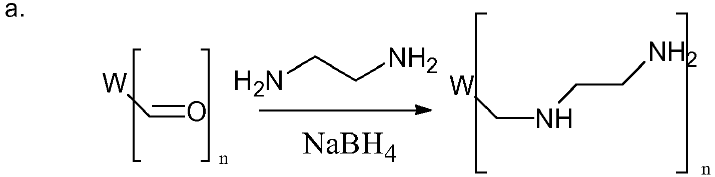 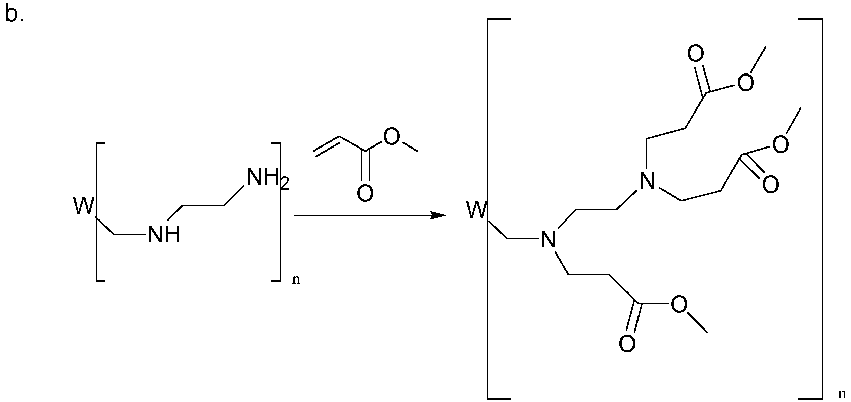 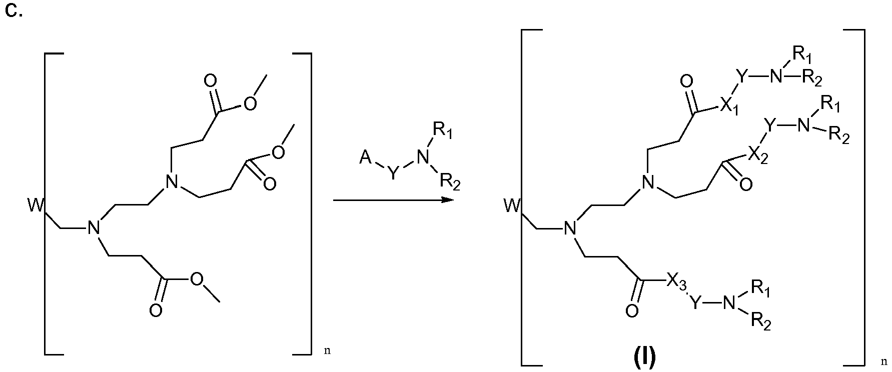 where:
W is selected from the group formed by any polymer or dendrimer derived from polyphenylenevinylene, polyphenyleneethylidene or hybrid of both, any conjugated organic compound that combines double bonds, triple bonds or a composition of both in its structure, any substitution with electron-donor or electro-attractor groups of the previous, any conjugated organic compound that alternates in its structure double bonds, triple bonds or a combination of both with aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous, any non-conjugated organic compound that contains aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous or any combination thereof.
A is selected from X₁, X₂ and X₃
X₁, X₂ and X₃ are the same or different and are selected from any heteroatom,
Y is selected from a saturated or unsaturated alkyl group (C₂-C₁₂) or cycloalkyl (C₃-C₈), cycloalkenyl, aryl or heteroaryl,
R₁ and R₂ are the same or different bound to a primary, secondary or tertiary amino group and are selected from hydrogen, saturated or unsaturated alkyl (C₁-C₁₂), and
n represents the number of branches and is a whole number selected from between 1 and 10.

Another fundamental aspect of the present invention relates to a process for the preparation of a compound of general formula (II) comprising the same stages for the preparation of the compound of general formula (I) starting from this last compound.

In another aspect, the present invention relates to the use of a compound of formula (I), (II) or (III) for the manufacturing of a medicament.

In another aspect, the present invention relates to the use of said medicament for the treatment and/or the prevention of diseases related to the nervous system such as neurodegenerative diseases, cerebrovascular accidents and diseases including processes, osteoporosis, peritoneal dialysis, diseases produced by viral infections.

Therefore, the compounds of the present invention can be used for the preparation of a medicament for the prevention or the treatment of diseases selected from the list comprising nervous system diseases such as neurodegenerative diseases (Parkinson's disease, dementia including Alzheimer's disease, Huntington's disease, demyelinating diseases such as multiple sclerosis and amyotrophic lateral sclerosis), cerebrovascular accidents (including the pathology derived from thrombosis and brain haemorrhage). It also includes in this section the treatment of tumours (especially prostate, lung and breast, without this list being limited to and exclusive of other types of tumoral pathology). This list also includes viral infections especially (although not exclusively) that causing Human Immunodeficiency Syndrome virus (HIV).

In another aspect, the present invention relates to the use of the compound of formula (I) or (II) for the preparation of a siRNA transfection kit in primary cultures of nerve cells, glia, and other primary cells such as hepatocytes (without the latter being exclusive of other primary cultures), and in tumoral, fibroblast, osteoblast cell lines (without being exclusive of other cell lines).

In another aspect, the present invention relates to the use of the compound of formula (I) or (II) in gene therapy as non-viral vector.

In another aspect, the present invention relates to the use of the compound of formula (I), or (II) for the preparation of probes, (radioactive or not) for the diagnosis of diseases by imaging techniques (Nuclear Magnetic Resonance, Single photon emission computed tomography or Positron emission tomography).

In another aspect, the present invention relates to the use of the compound of formula (I), (II) for the selective delivery of drugs, genetic material or cell line image probes. For this purpose, they must be in the form of salt and electrostatically bound to targeting groups. Said groups comprise, without being limiting in nature, antibodies to target cell proteins, analogues of molecules that make it possible to more effectively cross the hematoencephalic barrier such as transferrin or peptides that mimic their function, agonists of various receptors or signalling peptides that direct the molecule to the different intracellular compartments (nucleus, mitochondria, endoplasmic reticulum, lisosomes, endosomes, etc).

For their application in therapy, the compounds of formula (I) and (II), their isomers, salts, prodrugs or solvates, will be found, preferably, in a pharmaceutically acceptable or substantially pure form, i.e. that it has a pharmaceutically acceptable purity level excluding the normal pharmaceutical additives such as diluents and carriers, and not including material considered toxic at normal dosing levels.

The compounds described in the present invention, their pharmaceutically acceptable salts, prodrugs and/or solvates as well as the use of pharmaceutical compounds that contain them may be used together with other additional drugs to provide a combination therapy.

Said additional drugs may form part of the same pharmaceutical composition or, alternatively, they may be provided in the form of a separate composition for their simultaneous administration or not to that of the pharmaceutical composition comprising a compound of formula (I), or (II) or a pharmaceutically acceptable prodrug, solvate, derivative or a salt thereof.

Another preferred embodiment of the present invention relates to a pharmaceutical composition useful as a medicament for the prevention or the treatment of diseases selected from the list comprising nervous system diseases such as neurodegenerative diseases (Parkinson's disease, dementia including Alzheimer's disease, Huntington's disease, demyelinating diseases such as multiple sclerosis and amyotrophic lateral sclerosis), cerebrovascular accidents (including the pathology derived from thrombosis and brain haemorrhage). It also includes in this section the treatment of tumours (especially prostate, lung and breast, without this list being limited to and exclusive of other types of tumoral pathology). This list also includes viral infections especially (although not exclusively) that causing Human Immunodeficiency Syndrome virus (HIV), or, in general, which may benefit from the biological activities shown by the compounds described in the present invention, hereinafter pharmaceutical composition of the invention, comprising a compound, in therapeutically effective quantity, of formula (I), or (II), or mixtures thereof, a pharmaceutically acceptable salt, prodrug, solvate or stereoisomer thereof with a pharmaceutically acceptable carrier, adjuvant or vehicle, for administration to a patient.

The pharmaceutically acceptable adjuvants and vehicles that may be used in said compositions are the adjuvants and vehicles known by persons skilled in the art and typically used to prepare therapeutic compositions.

In the sense used in this description, the expression "therapeutically effective quantity" relates to the quantity of the agent or compound capable of developing the therapeutic action determined by their pharmacological properties, calculated to produce the desired effect and, in general, it will be determined, among other causes, by the typical characteristics of the compounds, including the age, condition of the patient, the severity of the alteration or disorder and the route and frequency of administration.

In another particular embodiment, said therapeutic composition is prepared in solid form or aqueous solution, in a pharmaceutically acceptable diluent. The therapeutic composition provided by this invention can be administered by any appropriate route of administration.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### BRIEF DESCRIPTION OF THE FIGURES

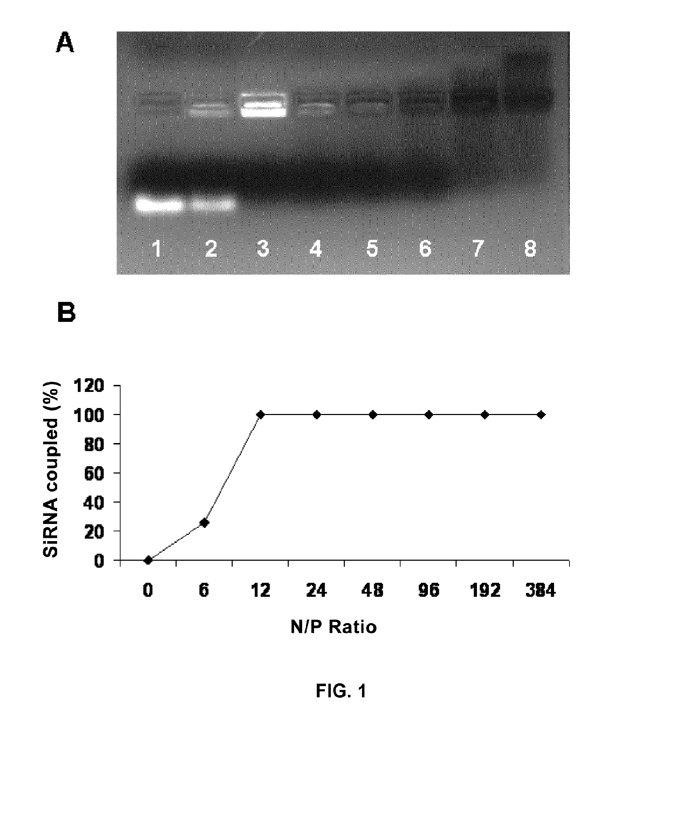

**FIG. 1****,** relates to the electrophoretic shift analysis of siRNA by the coupling to the compound of general formula (I). The numbers in (A) correspond to different N/P ratios (nitrogenated amines in compound of general formula (I)/phosphates in siRNA): (1) 1:0 (siRNA alone), (2) 6:1, (3) 12:1, (4) 24:1, (5) 48:1, (6) 96:1, (7) 192:1 and (8) 384:1. The densitometric analysis of the results of the gel shift experiment are shown in (B).

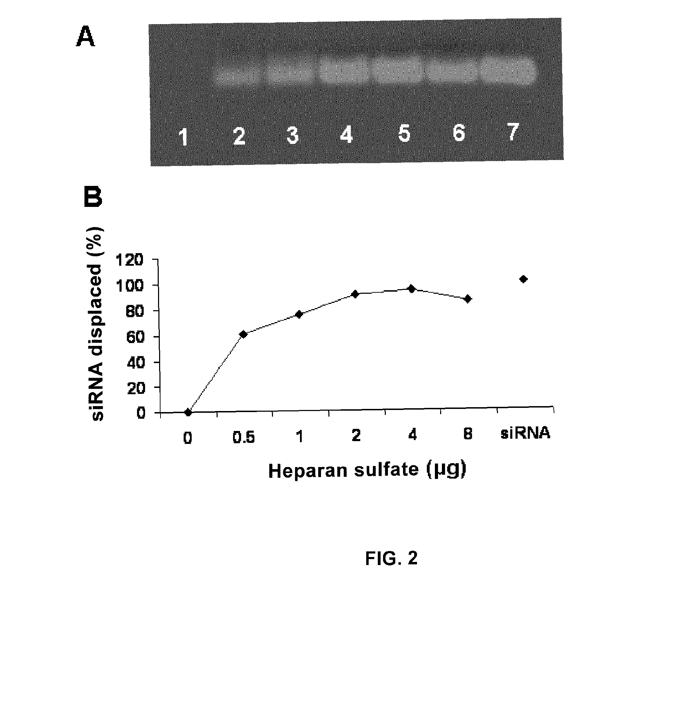

**FIG. 2** relates to the study of siRNA dissociation of the compound complexes of general formula (I)-siRNA by competition with heparan sulfate. The compound complexes of general formula (I)-siRNA with a ratio N/P of 12:1 were incubated during 1 hour at 37°C with increasing concentrations of heparan sulfate and the dissociation of the complexes was determined by means of electrophoresis in agarose gel. A. Agarose gel showing the siRNA released by increasing concentrations of heparan sulfate. The different lanes correspond to the incubation of the compound complex of general formula (I)-siRNA with the following concentrations of heparan sulfate (µg)/ compound of general formula (I) (µg): (1) 0,78, (2) 1,52; (3) 3,04; (4) 6,08; (5) 12,48; (6) 24,32 and (7) 0:0 (siRNA alone). The densitometric analysis of the results of the competition experiment with heparan sulphate are shown in (B).

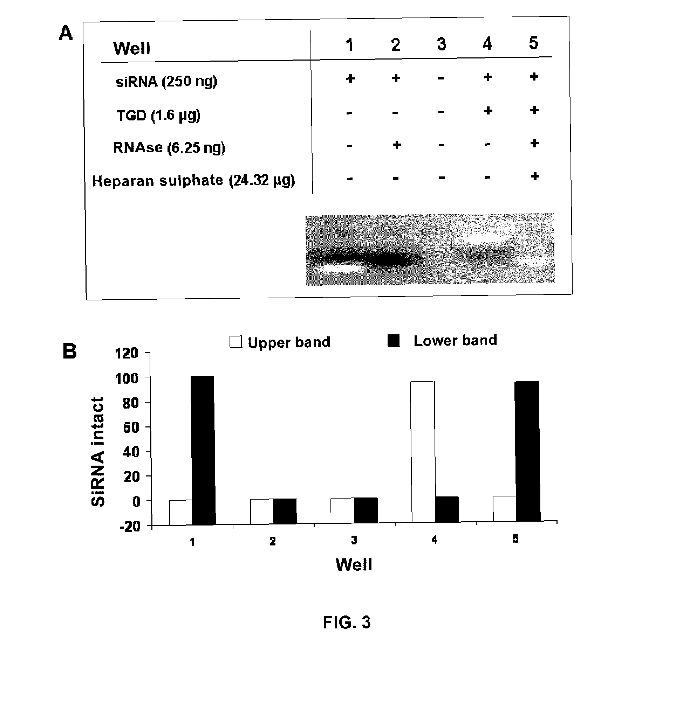

**FIG. 3** relates to the determination of the stability of the compound complex of general formula (I)-siRNA in the presence of RNAases. A. Effect of various treatments on the stability of the siRNA. B. Densitogram of A. The white bars represent the quantity of siRNA in the loading well and the black bars the quantity released by heparan sulfate at the end of the experiment.

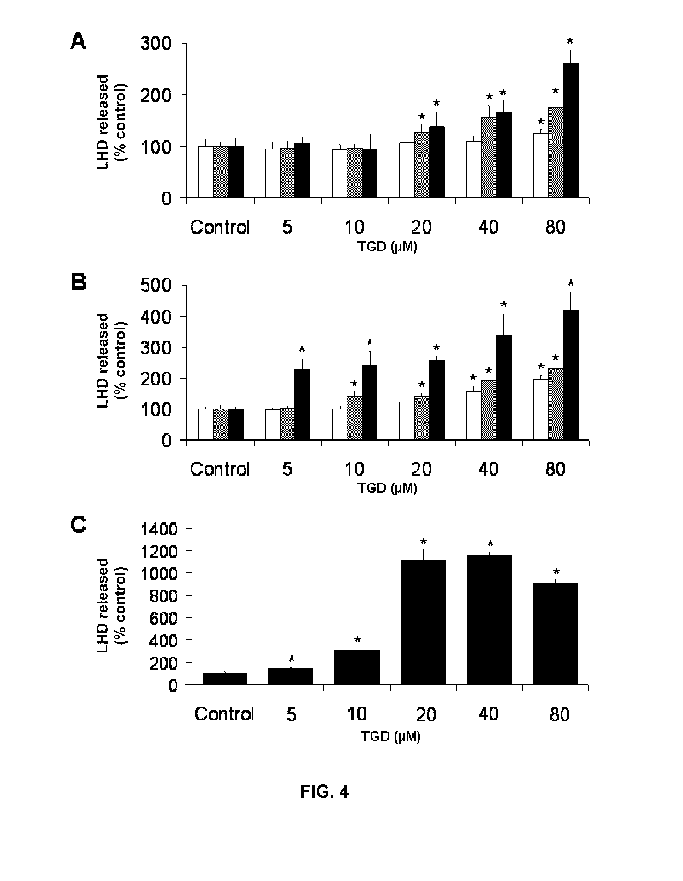

**FIG. 4** describes the toxicity study of compound of general formula (I) in PC12 cells (A), cerebellar granule neurons (B) and cortical neurons (C). The cells were treated with different concentrations of compound of general formula (I) (5 to 80 µM) during 24 hours (white bars), 48 hours (grey bars) or 72 hours (black bars). The cellular viability was assessed quantifying the percentage of LDH released into the culture medium. The data are expressed as mean (% control (% control) ± SEM, n=12. * p <0.05, compared with the control.

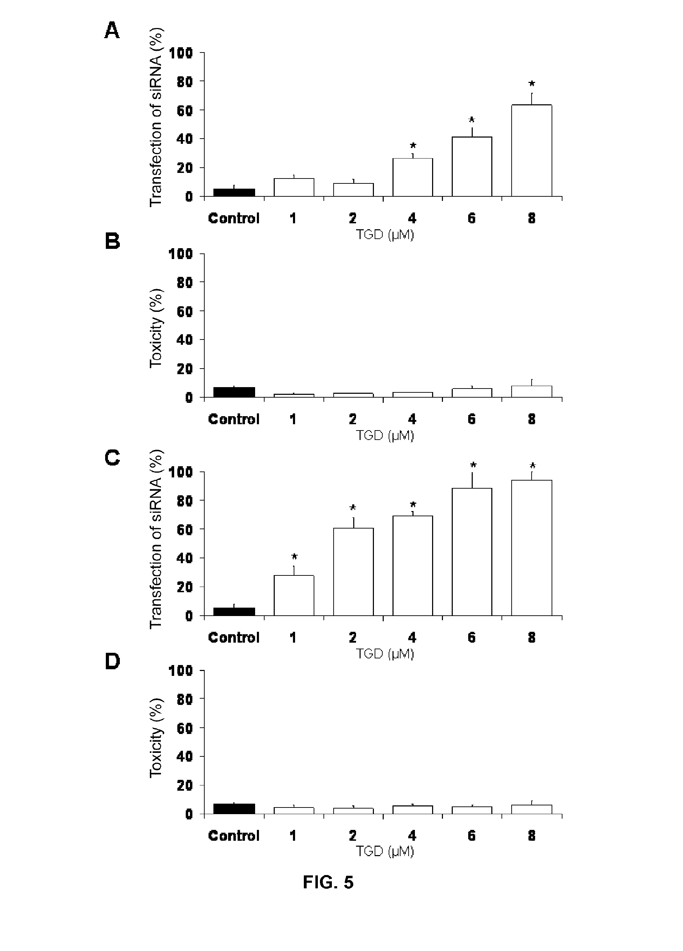

**FIG. 5** describes the study of the transfection and toxicity of compound of general formula (I) in PC12 cells. Quantification of the transfection of the compound complex of general formula (I)-fluorescent siRNA in PC12 cells (A, C) and of the toxicity produced by the complex (percentage of cells marked with propidium iodide) in this same cell type (B, D) by means of their study by flow cytometry. The complexes were formed with different concentrations of compound of general formula (I) and 100nM of fluorescent siRNA. The treatments lasted 24 hours (A, B) or 48 hours (C, D). The data are expressed as mean (% control) ± SEM, of a minimum of 3 different experiments. * p <0.05, compared with the control.

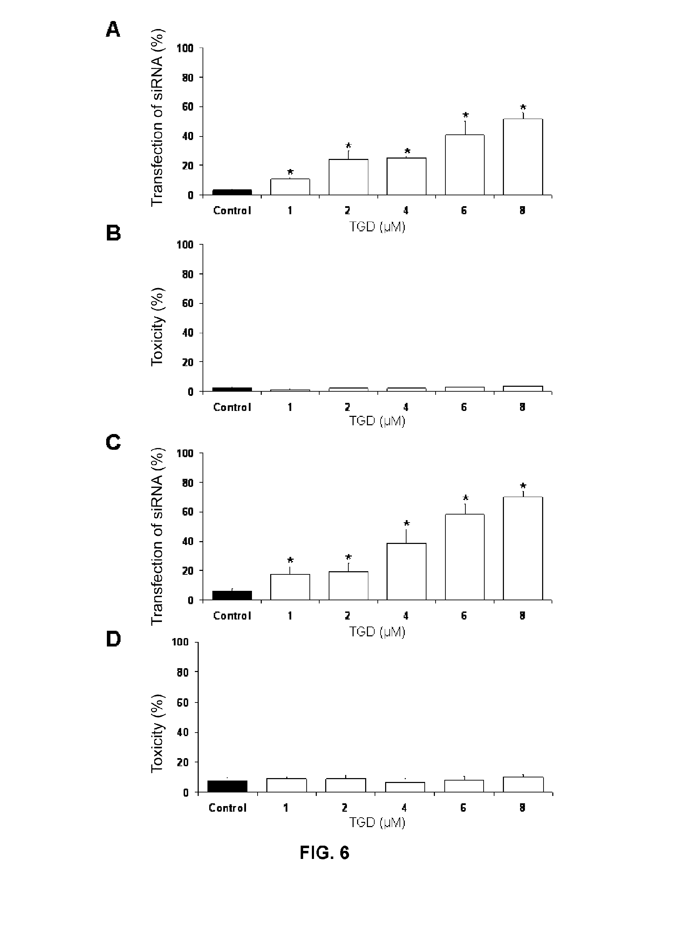

**FIG. 6** describes the study of the transfection and toxicity of compound of general formula (I) in LnCaP cells. Quantification of the transfection of the compound complex of general formula (I)-fluorescent siRNA in LnCaP cells (A, C) and of the toxicity produced by the complex (percentage of cells marked with propidium iodide) in this same cell type (B, D) by means of their study by flow cytometry. The complexes were formed with different concentrations of compound of general formula (I) and 100nM of fluorescent siRNA. The effects were studied after treatments of 48 hours (A, B) or 72 hours (C, D). The data are expressed as mean (% control) ± SEM, of a minimum of 3 different experiments. * p <0.05, compared with the control.

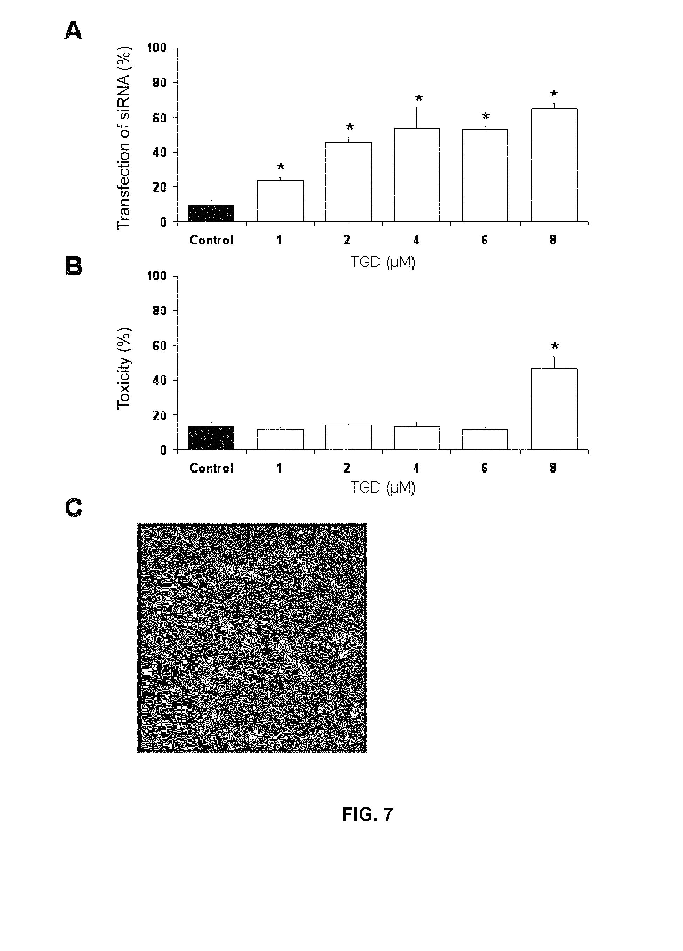

**FIG. 7** describes the study of the transfection and toxicity of compound of general formula (I) in cerebellar granule cells. Quantification of the transfection of the compound complex of general formula (I)-fluorescent siRNA, after 48 hours of treatment, in cerebellar granule neurons (A) and of the toxicity produced by the complex (percentage of cells marked with propidium iodide) in this same cell type (B) by means of their study by flow cytometry. The complexes were formed with different concentrations of compound of general formula (I) and 100nM of fluorescent siRNA. Microscopic study of the cerebellar granule neurons transfected with the complex formed by means of 4µM compound of general formula (I) and 100nM fluorescent siRNA (C). The data are expressed as mean (% control) ± SEM, of a minimum of 3 different experiments. * p <0.05, compared with the control.

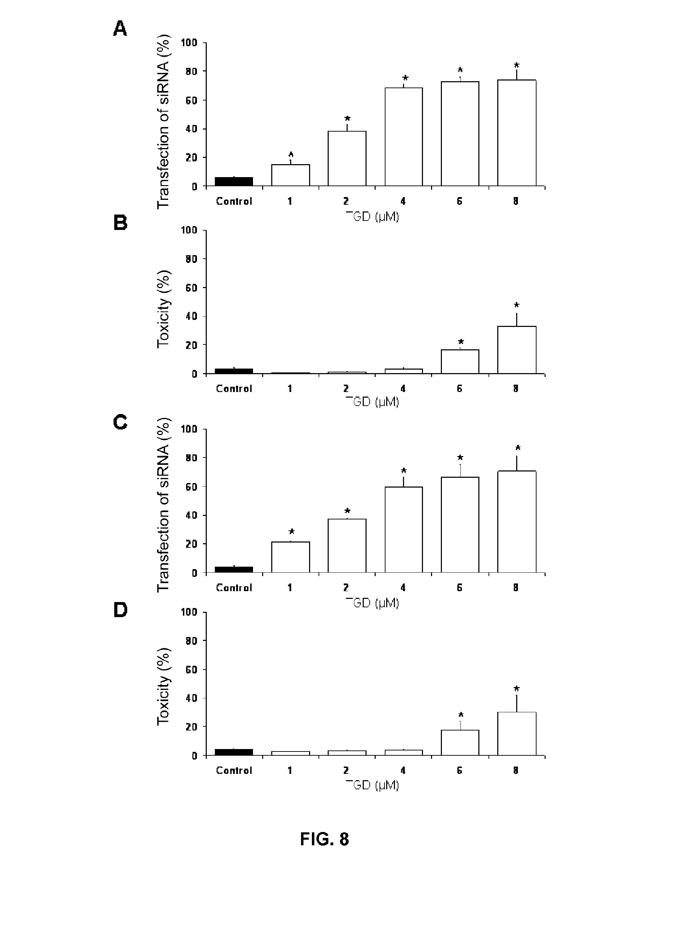

**FIG. 8** describes the transfection and toxicity of compound of general formula (I) in rat cortical neurons. Quantification of the transfection of the compound complex of general formula (I)-fluorescent siRNA in rat cortical neurons (A, C) and of the toxicity produced by the complex (percentage of cells marked with propidium iodide) in this same cell type (B, D) by means of their study by flow cytometry. The complexes were formed with different concentrations of compound of general formula (I) and 100nM of fluorescent siRNA. The effects were studied after treatments of 48 hours (A, B) or 72 hours (C, D). The data are expressed as mean (% control) ± SEM, of a minimum of 3 different experiments. * p <0.05, compared with the control.

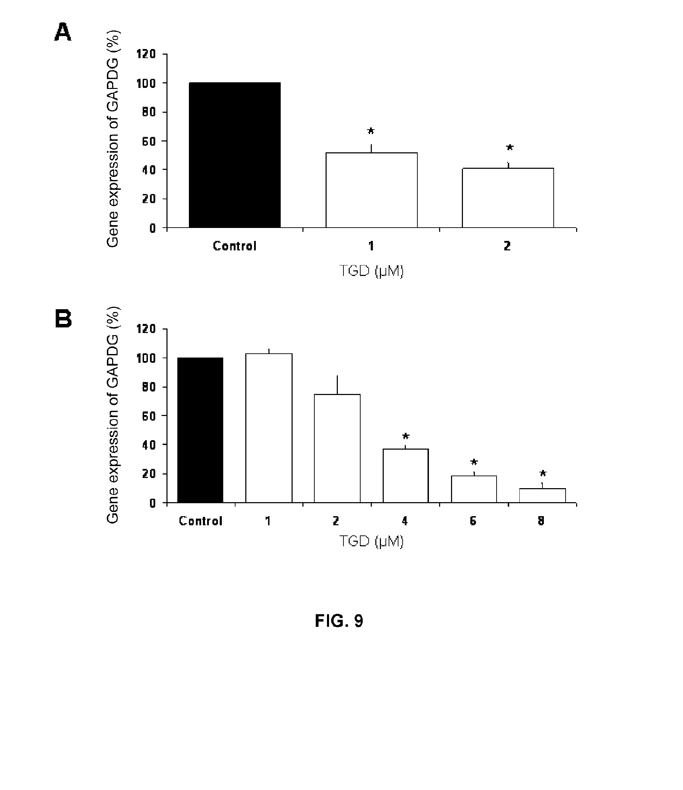

**FIG. 9** describes the study of the effect of the compound complex of general formula (I)-siRNA against GAPDH or SCRAMBLE (Control) in the gene expression of GAPDH in PC12 cells by means of real-time PCR. The quantification of the RNAm of GAPDH and β-action (endogenous control) was performed in transfected cells during 48 hours (A) or 72 hours (B). The data are expressed as mean (% control) ± SEM, of a minimum of 3 different experiments. * p <0.05, compared with the control.

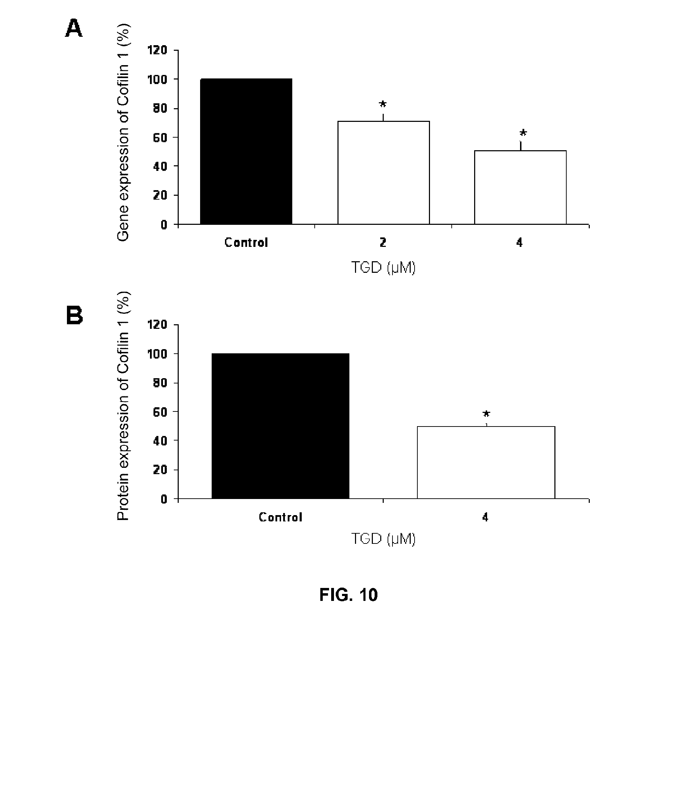

**FIG. 10** describes the study of the transfection during 48 hours of the compound complex of general formula (I)-siRNA against COFILIN 1 or SCRAMBLE (Control) in rat cerebellar granule neurons. A, by means of real-time PCR (A) the RNAm of COFILIN 1 and β-action (endogenous control) were quantified. B, Western blot analysis of the protein expression of COFILIN 1 and GAPDH (endogenous control). The data are expressed as mean (% control) ± SEM, of a minimum of 3 different experiments. * p <0.05, compared with the control.

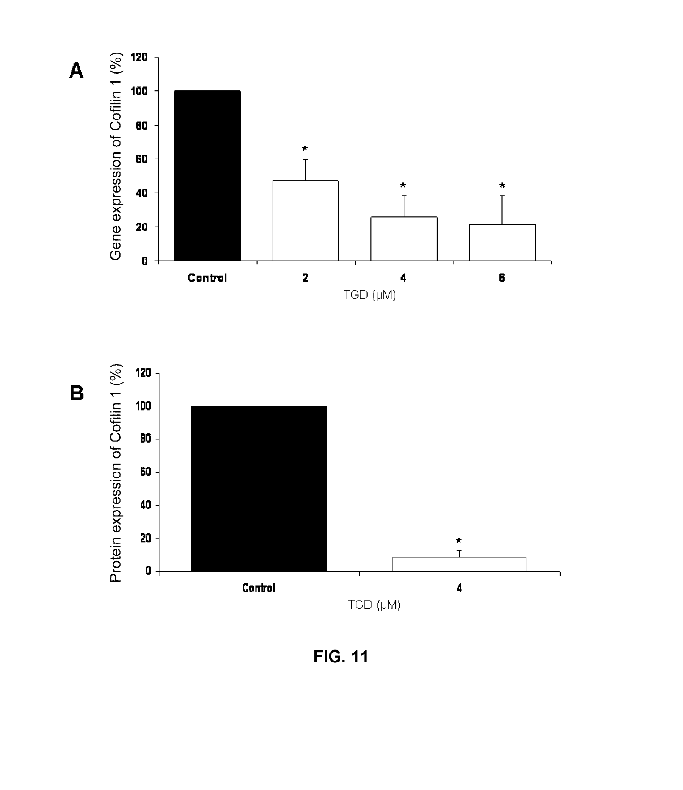

**FIG. 11** describes the study of the transfection during 48 hours of the compound complex of general formula (I)-siRNA against COFILIN 1 or SCRAMBLE (Control) in rat cortical neurons. A, by means of real-time PCR (A) the RNAm of COFILIN 1 and β-action (endogenous control) were quantified). B, Western blot analysis of the protein expression COFILIN 1 and GAPDH (endogenous control). The data are expressed as mean (% control) ± SEM, of a minimum of 3 different experiments. * p <0.05, compared with the control.

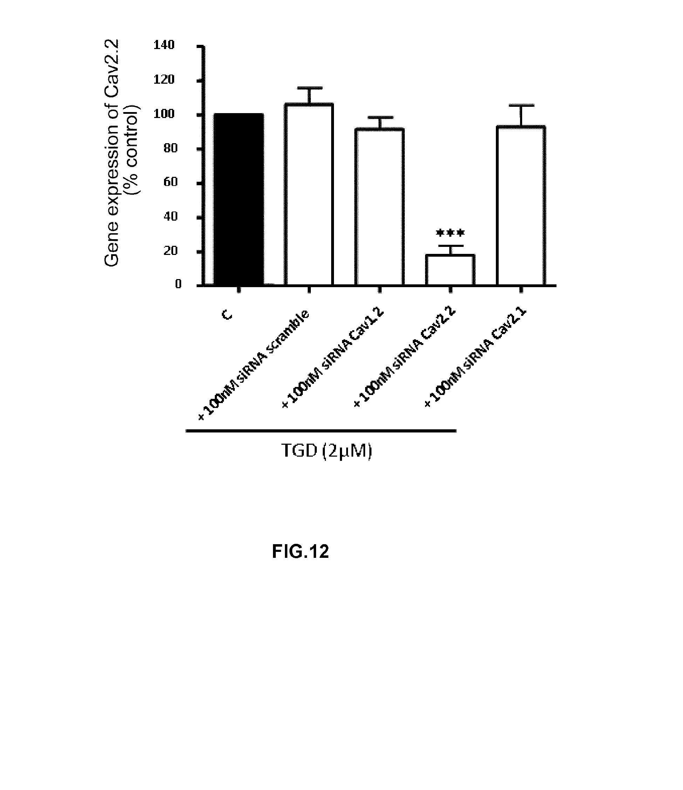

**FIG. 12** describes the study of the transfection during 48 hours of the compound complex of general formula (I)-siRNA (specific against the voltage-dependent calcium channel CaV2.2) against the voltage-dependent calcium channels CaV2.2, CaV1.2 and CaV2.1 or SCRAMBLE (Control) in bovine chromaffin cells. The RNAm was quantified for said channels by means of real-time PCR. The data are expressed as mean (% control) ± SEM, of a minimum of 3 different experiments.** * p <0,001, compared with the control.

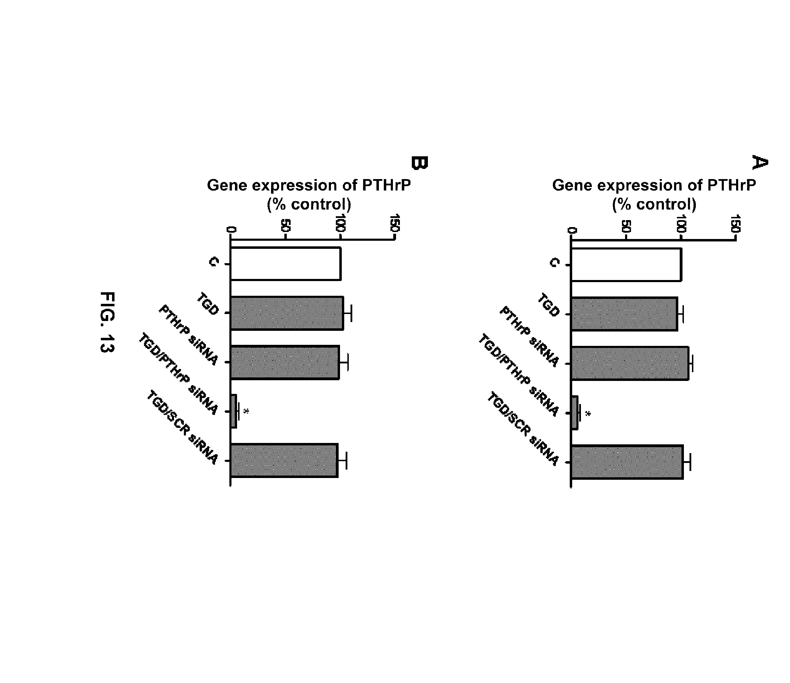

**FIG. 13** describes the study of the transfection during 48 hours of the compound complex of general formula (I)-siRNA against PTHrP or SCRAMBLE (Control) in human prostate cancer cells A. LnCaP and B. PC3. The RNAm of PTHrP was quantified by means of real-time PCR. The data are expressed as mean (% control) ± SEM, of a minimum of 3 different experiments. * p <0.05, compared with the control.

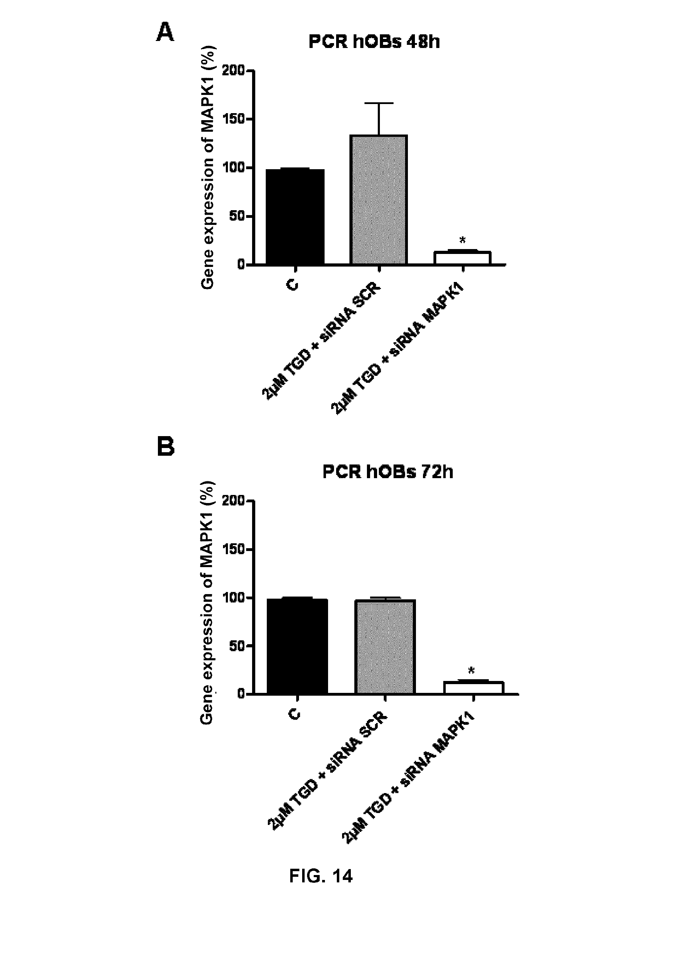

**FIG. 14** describes the study of the transfection during A. 48 hours or B. 72 hours of the compound complex of general formula (I)-siRNA against p42MAPK or SCRAMBLE (Control) in human osteoblast. The RNAm of p42MAPK was quantified by means of real-time PCR. The data are expressed as mean (% control) ± SEM, of a minimum of 3 different experiments. * p <0.05, compared with the control.

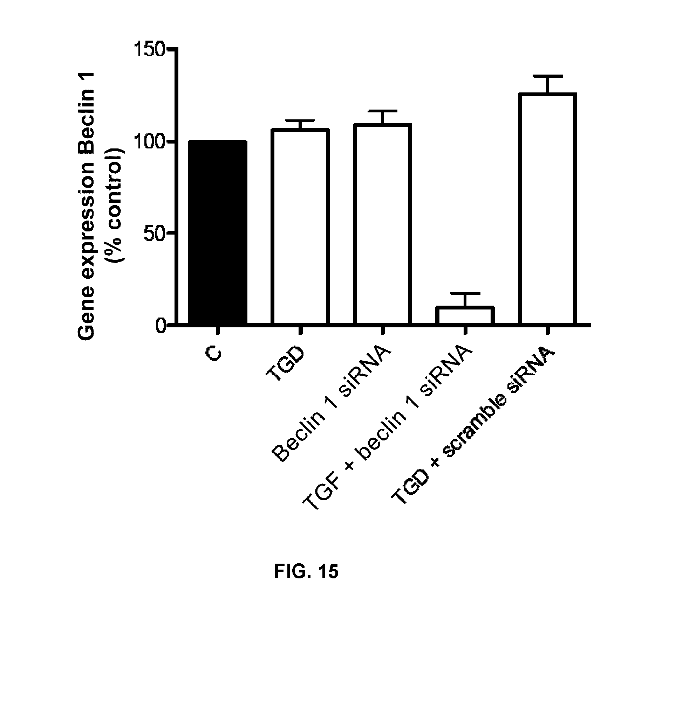

**FIG. 15** describes the study of the transfection during 48 hours of the compound complex of general formula (I)-siRNA against BECLIN 1 or SCRAMBLE (Control) in rat cortical neurons. The RNAm of BECLIN 1 was quantified by means of real-time PCR. The data are expressed as mean (% control) ± SEM, of a minimum of 3 different experiments. *** p <0.01, compared with the control.

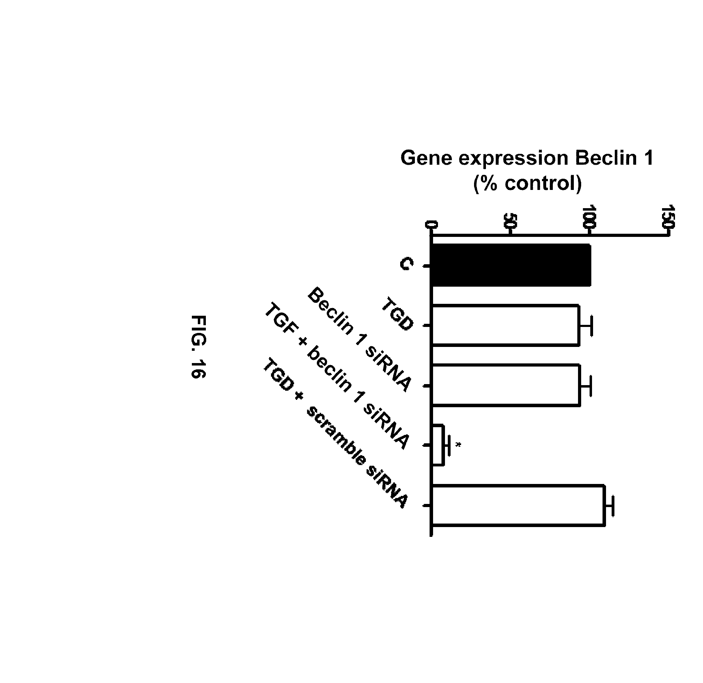

**FIG. 16** describes the study of the transfection during 48 hours of the compound complex of general formula (I)-siRNA against BECLIN 1 or SCRAMBLE (Control) in human prostate cancer cells, LnCaP. The RNAm of BECLIN 1 was quantified by means of real-time PCR. The data are expressed as mean (% control) ± SEM, of a minimum of 3 different experiments. *** p <0.01, compared with the control.

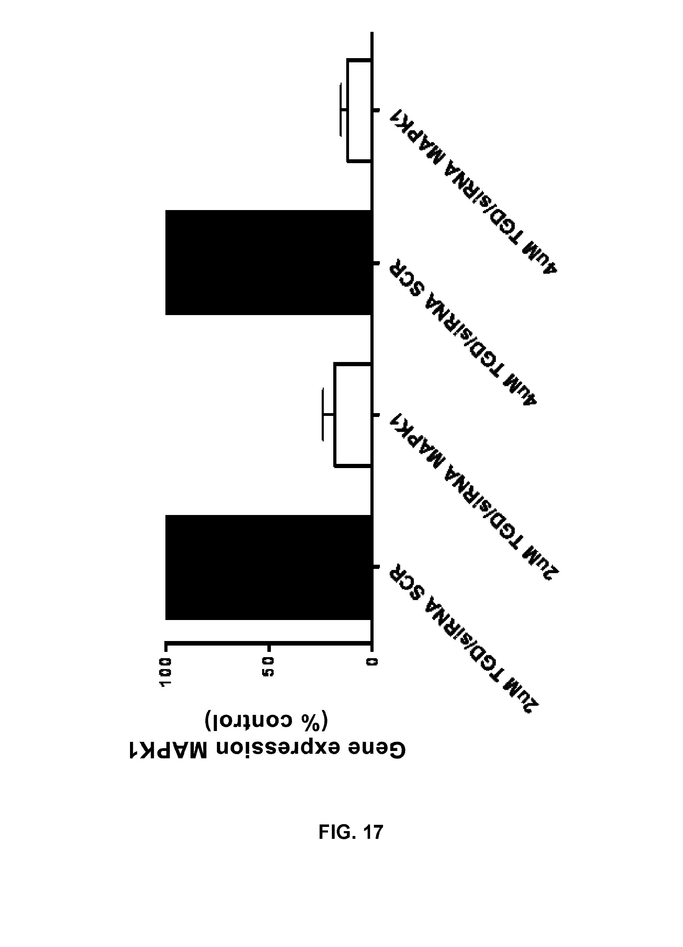

**FIG. 17** describes the study of the transfection during 96 hours of the compound complex of general formula (I)-siRNA against MAPK1 or SCRAMBLE (Control) in rat peritoneal cells in culture. The RNAm of MAPK1 was quantified by means of real-time PCR. The data are expressed as mean (% control) ± SEM, of a minimum of 3 different experiments. * p <0.05, compared with the control.

### EXAMPLES OF EMBODIMENT OF THE INVENTION

The following examples illustrate the present invention. However, these examples are not limitative. They are for information purposes and are in no case limiting of the methodologies used, which may be altered with the aim of achieving similar results.

In this specification, the symbols and conventions used in these processes, diagrams and examples are consistent with those used in the International System and contemporary scientific literature, for example, the Journal of Medicinal Chemistry. Unless indicated otherwise, all starting materials are obtained from commercial suppliers and were used without additional purification. Specifically, the following abbreviations can be used in the examples and throughout the specification: g (grams); mg (milligrams); Kg (kilograms); mL (millilitres); µL (microlitres); mmol (millimoles); mp (melting point); Hz (hertz); MHz (megahertz); δ (chemical displacement); ppm (parts per million); s (singlet); d (doublet); t (triplet); q (quartet); c (quintet); m (multiplet); J (coupling constant); NMR (Nuclear Magnetic Resonance); MS (mass spectrometry); ES (electrospray); *m*/*z* (mass/charge ratio); Anal. (Elementary Analysis); Yld (yield); TEA (triethylamine); CH₂Cl₂ (dichloromethane); CDCl₃ (deuterated chloroform); CD₃OD (deuterated methanol) DMSO (dimethylsulfoxide); i.p. (parenteral administration). All temperatures are expressed in °C (degrees Celsius).

### Example 1-

### PC12

The PC12 rat cells (adrenal gland; pheochromocytoma) were cultured in RPMI culture medium supplemented with 10% of heat-inactivated horse serum, 5% foetal bovine serum (FBS), 2mM glutamine, 100 U/ml penicillin and 100 µg/ml streptomycin, in 5% CO₂ at 37°C, according to the manual of the cell line source bank and previous publications (J Neurochem. 2007. 103: 1396-1407).

### SH5YSY

The SH-SY5Y cell line comes from a neuroblastome artificially cloned from a group of cells that expressed a characteristic phenotype (Cancer Res. 1978. 38: 3751-3757). This cell line is genetically feminine as the original line was established in 1970 from a biopsy of bone metastasis of a neuroblastome suffered by a 4-year old girl. The cells have an anomaly in chromosome 1, where a trisomy 1 q is found. The SH-SY5Y cells are known for being dopamine beta-hydroxylase active, acetylcholinergic, glutaminergic and adenosynergic. The cells are propagated by mitosis and by neurites extending to the peripheral areas. They were cultivated in a combination 1:1 of DMEM culture medium and HAM'S-F12 culture medium, supplemented with 10% FBS, 2mM glutamine, 0.5 mg/ml neomycin, 100 U/ml penicillin and 100 µg/ml streptomycin according to the manual of the cell line source bank.

### LnCaP

The LnCaP cells (ATCC CRL 1740) are a well-characterized cell line of human prostate carcinoma. They were cultivated in RPMI-1640 medium with 10% FBS, 2mM glutamine, and antibiotics (100 UI/ml penicillin and 100 µg/ml streptomycin), according to the manual of the cell line source bank and previous publications (Life Sci. 2009. 85: 421-430).

### PC3

The PC3 cells (ATCC CRL 1435) are a well-characterized cell line of human prostate carcinoma. They were cultivated in RPMI-1640 medium 10% FBS, 2mM glutamine, and antibiotics (100 UI/ml penicillin and 100 µg/ml streptomycin), according to the manual of the cell line source bank and previous publications (Life Sci. 2009. 85: 421-430).

### Cultures of rat peritoneal mesothelial cells.

The rat peritoneal mesothelial cells were isolated by enzymatic digestion in accordance with previously mentioned protocols (Perit Dial Int 1989; 9: 341-347). Briefly, female rats with a weight of 200-400 g of the Sprague-Dawley strain were sacrificed as established in Royal Decree 1201/2005, of 10 October, on the Protection of Animals used for Experimentation and other scientific purposes. The abdominal cavity was quickly opened and the abdominal wall (peritoneum and smooth muscle) was removed in sterile conditions. The mesothelial cells were separated from the inner surface of the abdominal wall by enzymatic digestion, incubating said surface with 199 culture medium (Sigma) and 1 mg/ml of collagenase A (Sigma) during 30 minutes at 37°C. After the incubation, the digested surface of the abdominal wall was scraped to completely release the adhered mesothelial cells. The mesothelial cells obtained were seeded in culture dishes for their growth and study, and were maintained in 199 medium supplemented with 10% foetal bovine serum, 100U/ml penicillin and 100µg/ml streptomycin at 37°C and 5% CO2 atmosphere. The medium was changed 24 hours after seeding and then every 2 days. After each culture, the presence of peritoneal mesothelial cells was confirmed by their morphological appearance and the expression of specific markers. For their study, cells were used between steps 3 and 6.

To perform the cell runs, the confluent cultures were washed twice with a saline phosphate buffer solution and were then exposed to 0.05% trypsin-EDTA (Gibco) until the cells completely came off. The effect of the trypsin was blocked with 199 medium supplemented with 10% foetal bovine serum. The cells were centrifuged at 900 r.p.m during 5 min at 20°C and were cultured as previously described.

### Culture of human osteoblasts

The human osteoblasts were obtained from the trabecular area of patients' bones (ages between 55-83 years) who had undergone hip surgery. All participants were informed before the surgery and gave their consent. The samples reached the laboratory in Locke solution at 4°C, from the operating theatre of the Hospital General Universitario de Albacete and were immediately processed. The femur head was deposited on a petri dish containing 10 mL of α-MEM (Invitrogen) medium with L-glutamine (2 mM), penicillin-streptomycin (1 %) and FBS (15 %) (complete medium) whereto fungizone was added (2.5 µg/ml). Explants of the trabecular zone were extracted and cut in small fragments (2-4 mm²), washing the femur head with a sterile syringe several times, using complete medium with fungizone. The medium, together with the trabecular bone explants were deposited in a sterile falcon, which was mechanically stirred and centrifuged at 10g during 3 minutes. The supernatant was transferred to another falcon and was again centrifuged at 1500 g during 5 minutes. Then, the pellet obtained was resuspended in 6 mL of complete medium, and the cells were seeded in a P6 dish. The cells were incubated at 37°C with a 5% CO₂ atmosphere. After 24 hours, a change of medium was performed and the medium was then replaced every 2-3 days. Once confluence had been reached, a *splitter* 1:3 was carried out, and successive *splitters* were performed until reaching step 3. At this point, the culture medium was supplemented with ascorbic acid (50 µg/µl) and β-glycerolphosphate (10 mM), to induce osteoblastic differentiation.

The cells were characterized as osteoblasts by means of determining alkaline phosphatise and measuring the expression levels of mRNA of RUNX2 and OSX, by means of q-PCR, using a cell line of human fibroblasts as negative control.

### Primary culture of bovine chromaffin cells.

The chromaffin cells of the adrenal medulla were isolated from the bovine adrenal glands, extracted from the animals in a local slaughterhouse and transported to the laboratory at 4°C in *Locke solution* (in mM: 154 NaCl; 5.6 KCI; 3.6 NaHCO₃; 5.6 glucose; 5 Hepes; pH 7.4), supplemented with 50 UI/ml penicillin (Sigma, St. Louis, MO), 50 µg/ml streptomycin (Sigma) and 1mM glutamine (Invitrogen, Carlsbad, CA).

The culture protocol followed was that of digestion of the adrenal medulla with collagenase described by *Livett et al.* in 1984 (Livett, B.G., 1984) with the modifications introduced by *Moro et al.* in 1990 (Moro, M.A. et al., 1990). In summary, first the periadrenal fatty tissue was removed and 3.5 ml of enzymatic solution with 0.25% collagenase (Roche Applied Sciences, Indianapolis, IN) and 5% bovine serum albumin (BSA, Sigma) were injected through the adrenolumbar vein (or central medullar), incubating the glands at 37°C during 45 minutes. Then, the medulla was extracted, it was cut into small pieces (less than 1 mm³) to produce minimum damage to the cells and it was again incubated in collagenase enzymatic solution at 37°C during 30 minutes.

Then**,** the adrenergic and noradrenergic chromaffin cells were purified by means of a Percoll gradient, and after successive washings with *Locke* solution they were resuspended in DMEM culture medium (Invitrogen) supplemented with 10% foetal bovine serum (FBS, Invitrogen), 1mM glutamine and antibiotics (50 UI/ml penicillin and 50 µg/ml streptomycin). The chromaffin cells were seeded in dishes or glass plates previously treated with 50 µg/ml of poly-L-lysine (poly-L, Sigma) at a concentration of 100,000 cells/cm². The cells were maintained at 37°C in an incubator with atmosphere saturated with water and 5% CO₂. In the experiments, cells of 1 to 5 days culture were used.

### Primary cultures of rat cerebellar granule neurons

The culture of cerebellar granule neurons were obtained in accordance with previously described protocols (J Neurochem. 2007. 103: 1396-407; Brain Res Dev Brain Res. 1991. 63: 1-12), with small modifications. Briefly, 7 day old rats of the Spragle-Dawley strain were quickly decapitated and their brains were carefully removed. We separate the cerebellum aseptically, we remove the meninges and the brain was cut into pieces of approximately 0.4 mm. Then, the tissue was exposed to trypsin and DNAse in a culture medium free of calcium and magnesium and they were seeded in culture dishes pretreated with poly-lysine. The cells were cultivated in BME medium supplemented with 24.5 mM potassium, 2mM glutamine, 10% FBS and 50µg/ml gentamycin. After 24 hours, Ara-C (cytosine arabinoside) was added to the medium to obtain a final concentration of 10 µM to reduce the growth of astrocytes. The cells were used not before 7 days after culture, which is the time they need to complete differentiation.

### Primary cultures of rat cortical neurons

The primary culture of cortical neurons was performed in accordance with the previously described methodology (Eur J Neurosci. 2001.; 13: 1469-78). The frontolateral cortical lobes were dissected in 17-day foetuses of female rats of the Spragle-Dawley strain and they were mechanically dissociated in HBSS. The cortical lobes were titrated pipetting ten times with a Pasteur pipette. After centrifuging for 5 minutes at 800xg, the cells were resuspended in a Neuobasal culture medium supplemented with B27, 2mM of glutamine, 100 U/mL penicillin and 100 µg/mL streptomycin. The cells were seeded in culture dishes pretreated with poly-lysine and they were used not before 7 days after the culture, which is the time they need to complete differentiation and for glutamate receptors to appear.

### Formation of the compound complexes of general formula (I)-siRNA

The compound complexes of general formula (I)-siRNA were formed by mixing equal quantities of volume of the solution that contained compound of general formula (I) and of that containing the siRNA (Org Biomol Chem. 2007. 5: 1886-1893; Pharm Res. 2009. 26: 1181-1191)*,* and incubating the mixture under stirring during 30 minutes at ambient temperature. Both molecules were dissolved in DEPC water (free from RNAses).

Gel shift experiments, exclusion with heparin and protector effect of the compound complex of general formula (I)-siRNA to the action of RNAses

The shift in agarose gel was used to ascertain the N/P ratio (nitrogenated amines in compound of general formula (I)/phosphates in siRNA) adapted to obtain the greatest efficacy of bonding possible between both molecules (Mol Biol Rep. 2009. 36: 1083-1093; Am J Med Genet B Neuropsychiatr Genet. 2008. 147B: 769-777). The mixing of different concentrations of compound of general formula (I) and of 250ng of siRNA was tested. The mixture is run for 15 minutes at 60V in a 1.2% agarose gel with 0.017% ethydium bromide. The gels were photographed and the bands were quantified with a suitable image analysis system (Quantity One).

The heparin shift experiments were performed to assess the bonding force between compound of general formula (I) and siRNA. The complexes were prepared with a N/P ratio of 12:1, which was considered optimal to guarantee a complete coupling of the siRNA with the compound of general formula (I). Then, they were incubated with 0.78; 1.52; 3.04; 6.08; 12.48 and 24.32 µg of heparan sulfate at 37°C during 1 hour. The solutions were run in an agarose gel in the same conditions as the ratio test of compound of general formula (I)-siRNA described above.

For the experiments to assess the protector effect of the compound complex of general formula (I)-siRNA, the complexes with a N/P ratio of 12:1 were incubated, instead of with different quantities of heparan sulfate, with 0.25% RNase A during 30 minutes at 37°C. Then, the samples were incubated with excess heparan sulphate to guarantee a complete release of the siRNA from the complex. The percentage of intact siRNA was analysed by electrophoresis in agarose gels in the same conditions as the previously described experiments.

A minimum of 2 experiments were performed for each one of the aforementioned tests.

### Citotoxicity studies

Tests to assess the toxicity of the compound of general formula (I)- were performed in different cell types, determining the activity of the enzyme lactate dehydrogenase (LDH) (Pharm Res. 2009. 26: 1181-1191)*.* For this purpose, the cells were seeded in 24-well plates and they were exposed to solutions with different concentrations of compound of general formula (I)-(5-80 µM) to perform concentration-dependent toxicity curves during 24, 48 or 74 hours. The toxic effects were assessed by measuring the rupture of the cell membrane and consequent release of the LDH to the supernatant through the CytoTox96® kit (Promega). The cells were mechanically removed, they were washed with PBS and were centrifuged at 10,000 rpm during 10 minutes. The absorbance of the lysate and of the cellular supernatant was measured using a microplate spectrophotometer at a wavelength of 490 nm.

The toxicity of the treatments with the compound complexes of general formula (I)-siRNA, using different concentrations of compound of general formula (I)- (1-8 µM) in combination with 100 nM of siRNA, was studied by flow cytometry. For this purpose, after the treatments, the cells were incubated with propidium iodide (0.5 mg/mL) during at least 1 hour at 37°C in the dark. Then, the cells were trypsinized and they were analysed in a flow cytometer (FACSCalibur, Becton-Dickinson, Franklin Lakes, NJ, USA). From the evaluation of 10,000 cells per experimental condition, the percentage of cells with damaged cytoplastic membrane was calculated (positive propidium iodide) (Weber N et al., 2008; Perumal OP et al., 2008).

### Study of the percentage translocation of the compound complex of general formula (I)-siRNA to the cell interior

After 24-72 hours with the cells in the presence of the compound complexes of general formula (I)-siRNA, using 100nM of fluorescent siRNA to perform them, the conditioned media were collected and the cells were trypsinized and washed with PBS. The total cells - live and dead - present in the resulting suspension on combining the cell trypsinate and the conditioned medium were analysed in a flow cytometer (FACSCalibur, Becton-Dickinson, Franklin Lakes, NJ, USA). From the evaluation of 10,000 cells per experimental condition, the percentage of cells transfected with fluorescent siRNA was calculated (J Control Release. 2008. 132: 55-64; Biomaterials. 2008. 29: 3469-76).

The translocation of the compound complex of general formula (I)-siRNA is also studied by confocal microscopy. For this purpose, they were seeded on slides and treated in the same way as the previous samples. The cells treated with fluorescent siRNA, alone or forming compound complexes of general formula (I)-siRNA, were viewed and photographed in a confocal microscope (Nikon Eclipse TE200) using the suitable wavelength for the excitation of the fluorophore with which the siRNA is marked (Pharm Res. 2009. 26: 577-86). The results served to determine the percentage of positive cells for the intracellular transfection of siRNA.

### Study of the gene silencing by chain reaction of the real time polymerase chain reaction (real-time PCR)

The total cellular RNA was isolated by means of a standard method with guanidine-phenol-chloroform thiocyanate (TriPure Isolation Reagent, Roche Applied Sciences, Indianapolis, IN). The RNA was transformed into cDNA and this was used to perform the real-time PCR. We use real-time PCR to study the silencing of the different genes by means of 100 nM of siRNA vehicularized with different concentrations of compound of general formula (I). The beta-actin gene was used as a reference gene for all the real-time PCR experiments. The reaction was performed using standard processes for the StepOnePlus Real-Time PCR System (Applied Biosystems).

In each experiment, the mean of the cycle threshold (C_{T}) was calculated of each of the triplicates of each one of the genes studied and of the gene used as reference, thus being able to compare the gene expression of the different treatments (Pharm Res. 2009. 26: 577-86.; Cancer Res. 2007. 67: 8156-8163).

### Evaluation of the degree of protein silencing by Western blot

The cell extracts were obtained by lysating in 50 mM Tris-HCl, pH 7.4, 150 mM NaCl, 1 mM EDTA, 1% Triton X-100, 1% sodium deoxycholate and 0.1% SDS and protease inhibitors (5µg/ml aprotinin and 1mM PMSF). Once lystated, the cells were centrifuged at 13,000 r.p.m. during 15 minutes. The protein concentration present in the supernatant was determined by Bradford's method (Pierce; Rockford, IL, USA), using bovine serum albumin as standard. The samples containing 40 µg of total protein were applied in each well of polyacrylamide gel (10-15%) according to the protein to study. The gels were transferred by electrophoresis to nitrocellulose membranes using a semi-dry blotter. The proteins bound to nitrocellulose were viewed with Poinceau, followed by blocking with TTBS (50 mM Tris, pH 7.5, 200 mM NaCl, 0.1% Tween) with 5% of skimmed milk and, later, they were incubated with the corresponding primary antibody throughout the night at 4°C. After washing in TTBS, the secondary antibody was applied during 1 hour at ambient temperature. The detection was performed by chemiluminescence. The intensity of the bands was analysed by levels of grey with an appropriate image analysis system (Quantity One) (Pharm Res. 2009. 26: 1181-1191).

### Results

Gel shift experiments, exclusion with heparin and protector effect of the compound complex of general formula (I)-siRNA to the action of RNAses.

2.5 µl of 40µM siRNA is incubated, during 30 minutes at ambient temperature, in a final volume of 50 µl in an Eppendorf tube, to give a final concentration of 2 µM containing a total of 6.02 x 10¹¹ negative charges together with a concentration of the dendrimer compound of general formula (I) sufficient to give a nitrogen ratio of the dendrimer/phosphate of siRNA (N/P) of 6:1 and the quantity of dendrimer is increased, the quantity of siRNA remaining fixed until reaching a P/N ratio of 384:1 (figure 1). After the incubation, the samples were subjected to electrophoresis in 1% agarose gel during 15 minutes at 60V, it is incubated with ethydium bromide and the siRNA is viewed with ultraviolet light. As can be observed in figure 1, at a P/N ratio of 12:1 (concentration of compound of general formula (I) of 22 µM) all the siRNA is fixed by the dendrimer and is not displaced in the gel.

### Example 2. Synthesis of a compound of general formula (I)

Schlenk standard techniques are used in all those reactions that require an inert atmosphere (argon).

For the reactions in inert atmosphere, both tetrahydrofuran (THF) and dichloromethane (CH₂Cl₂), of CHROMASOLV^{®} grade (Aldrich) were previously purified using a solvent purification system manufactured by Innovative Technology. The ethylenediamine was dried at ambient temperature on calcium hydride during 2 hours, it was filtered and distilled at reduced pressure (30-40 mm of Hg), being stored on a molecular screen (4 A). The MeOH was dried on calcium oxide, it was filtered and distilled being stored on molecular screen (4 A). The other solvents were used without prior purification.

### 2.1. Synthesis diagram:

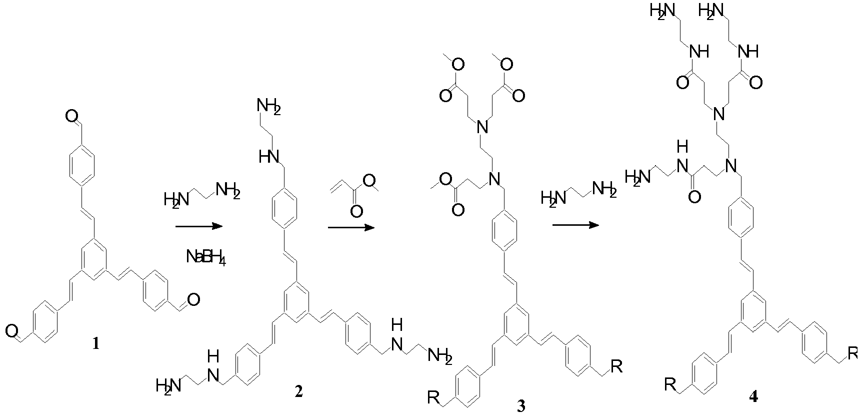

Compound 1 is prepared in accordance with Synthesis, Characterization, and Optical Response of Dipolar and Non-Dipolar Poly(phenylenevinylene) Dendrimers. J. J. Org. Chem. 2001, 66(17), 5664-5670.

### 2.2. Synthesis of Compound 2:

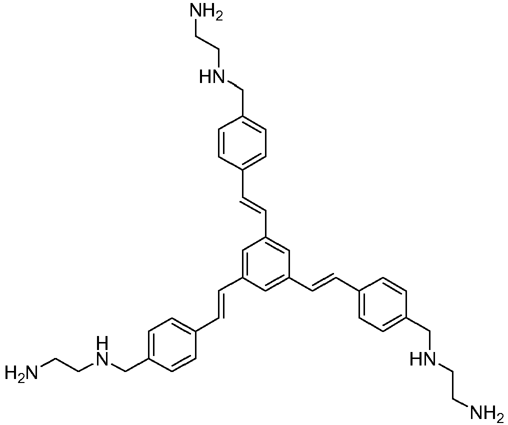

The ethylenediamine (8.6 mL, 128 mmol) was added drop by drop to a solution containing trialdehyde 1 (300 mg, 0.64 mmol) in anhydrous CH₂Cl₂ (50 mL) with dry molecular screen, under argon. The reaction was stirred during 30 minutes and the molecular screen was filtered. In first place, the sodium borohydride (145 mg, 3.84 mmol) was added to the solution, under argon, then 10 mL of anhydrous MeOH. It was left to react for two hours. Then, the solvent was eliminated under vacuum and 3 cycles of dissolution-evaporation were performed with a mixture of toluene/MeOH in 9:1 (10 mL) ratio and a final one with 10mL of MeOH. Then, the resulting white solid is centrifuged with 3 fractions of water of 5 mL, and the solid is dissolved in CHCl₃ and dried with MgSO₄ during 2 hours. After filtering and eliminating the solvent under vacuum, the compound was purified by means of hot washing with THF obtaining 351 mg (0.58 mmol) (91 %) of the desired compound as a white-coloured solid.

¹H-NMR (CD₃OD, 500 MHz) δ: 2.73 (t, 6H, J=6 Hz, -CH₂-NH₂), 2.83 (t, 6H, J=6 Hz, -NH-CH₂-), 3.79 (s, 6H, -CH₂-NH-), 7.21 (A of ABq, 3H, J = 16.5 Hz, 3xCH=), 7.29 (B of ABq, 3H, J = 16.5 Hz, 3xCH=), 7.37 (A of ABq, 6H, J = 7.5 Hz, 6xArH), 7.58 (B of ABq, 6H, J = 7.5 Hz, 6xArH), 7.63 (s, 3H, ArH).
¹³C NMR and DEPT (CD₃OD, 125 MHz) δ: 140.27 (C), 139.62 (C), 137, 85 (C), 129.98 (CH), 129.92 (CH), 129.30 (CH), 127.78 (CH), 124.90 (CH), 53.82 (-CH₂-NH-), 50.59 (-NH-CH₂-), 41.09 (-CH₂-NH₂).

### 2.3. Synthesis of Compound 3:

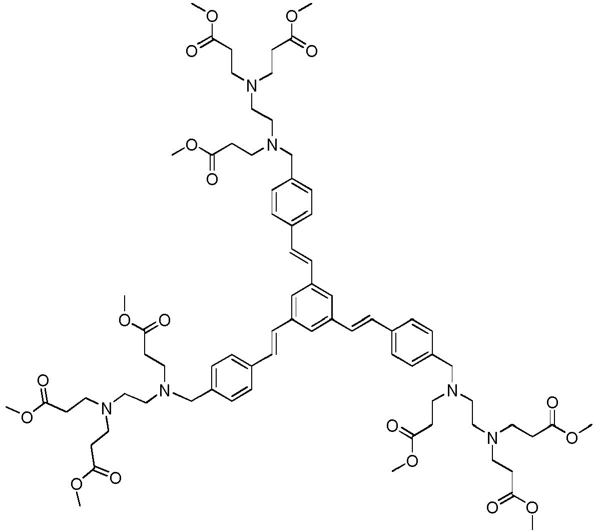

A solution of compound 2 (0.250 g, 0.416 mmol) in MeOH (20 mL) was cooled to 0°C in an ice bath. Once this temperature is achieved, the methyl acrylate is added slowly (3.06 g, 35.53 mmol) and it was left to react at ambient temperature during 4 days. Then, the solvent was eliminated with an excess of methyl acrylate under vacuum, the compound was purified by means of hot washing with THF giving rise to 0.324 g (57%) of the desired produce as a white-coloured solid.

¹H-NMR (CDCl₃, 500 MHz) δ: 2.41 (t, 12H, *J*=7 Hz, -CH₂-CO-), 2.49 (t, 6H, *J*=7 Hz, -CH₂-CO-), 2.54 (s, 12H, -N-CH₂-CH₂-N-), 2.74 (t, 12H, *J*=7 Hz, -N-CH₂-), 3.61 (s, 6H, -CH₂-NH-), 3.65 (s, 18H, -OCH₃), 3.67 (s, 9H, -CH₂-NH-), 7.14 (A of ABq, 3H, *J* = 16.5 Hz, 3xCH=), 7.20 (B of ABq, 3H, *J* = 16.5 Hz, 3xCH=), 7.31 (A of ABq, 6H, *J* = 8.5 Hz, 6xArH), 7.50 (B of ABq, 6H, *J* = 8 Hz, 6xArH), 7.55 (s, 3H, 3xArH).

¹³C NMR, DEPT and gHSQC (CDCl₃, 125 MHz) δ: 173.01 (-CO-), 172.94 (-CO-), 139.16 (C), 138.08 (C), 135.65 (C), 129.05 (3xCH=, 6xArH), 127.88 (3xCH=), 126.42 (6xArH), 123.72 (3xArH), 58.69 (-CH₂-NH-), 52.12 (-N-CH₂-CH₂-N-), 51.93 (-N-CH₂-CH₂-N-), 51.52 (CH₃), 49.50 (-CH₂-NH-), 49.63 (-N-CH₂-), 32.66 (-CH₂-CO-), 32.52 (-CH₂-CO-).
IR ν: 1734 cm⁻¹ (-CO₂⁻).
MALDI-TOF m/e: 1375.43 (M+H)⁺ and 1397.45 (M+Na)⁺

### 2.4. Synthesis of Compound 4: (IR8)

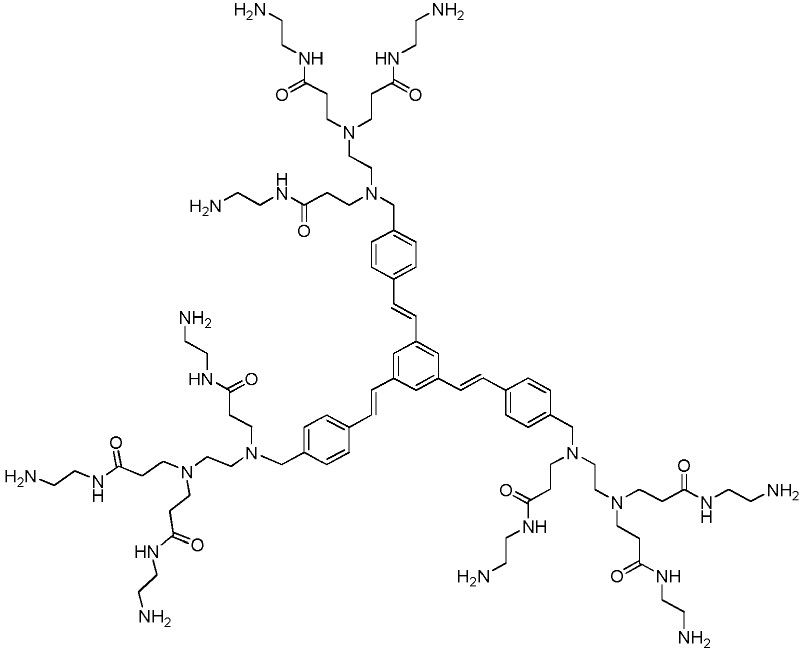

A solution of compound 3 (324 mg, 0.236 mmol) in ethylenediamine (10 mL) was stirred 48 hours at ambient temperature. Then, the solvent was eliminated in vacuum and 3 cycles of dissolution-evaporation were performed with a mixture of toluene/MeOH in 9:1 (10 mL) ratio and a final one with 10mL of MeOH. The compound was purified by means of hot washing with THF and 384 mg (85%) of compound 4 were obtained as a white solid.

## Claims

1. Compound of general formula (I) 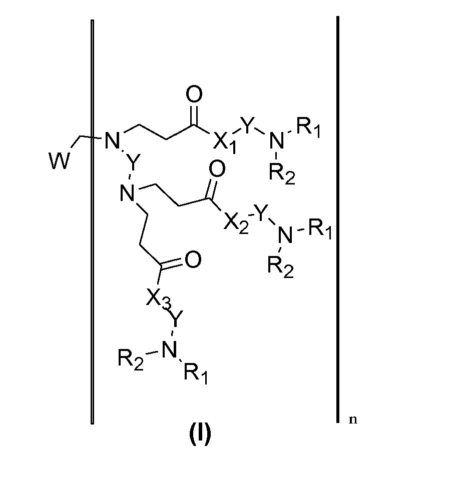 or a salt, prodrug or solvate thereof;
wherein:
W is selected from the group formed by any polymer or dendrimer derived from polyphenylenevinylene, polyphenyleneethylidene or hybrid of both, any conjugated organic compound that combines double bonds, triple bonds or a composition of both in its structure, any substitution with electron-donor or electro-attractor groups of the previous, any conjugated organic compound that alternates in its structure double bonds, triple bonds or a combination of both with aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous, any non-conjugated organic compound that contains aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous or any combination thereof;
X₁, X₂ and X₃ are the same or different and are selected from any heteroatom,
Y is selected from a saturated or unsaturated alkyl group (C₂-C₁₂) or cycloalkyl (C₃-C₈), cycloalkenyl, aryl or heteroaryl,
R₁ and R₂ are the same or different bound to a primary, secondary or tertiary amino group and are selected from hydrogen, saturated or unsaturated alkyl (C₁-C₁₂), cycloalkyl (C₃-C₈), cycloalkenyl, aryl or heteroaryl,
n represents the number of branches and is a whole number selected from between 1 and 10.

2. Compound of general formula (II) according to claims 1 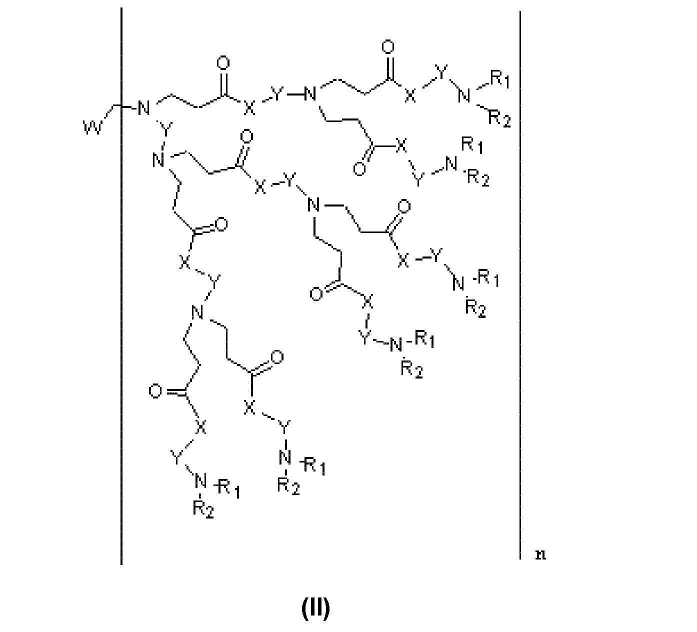 or a salt, prodrug or solvate thereof;
wherein:
W is selected from the group formed by any polymer or dendrimer derived from polyphenylenevinylene, polyphenyleneethylidene or hybrid of both, any conjugated organic compound that combines double bonds, triple bonds or a composition of both in its structure, any substitution with electron-donor or electro-attractor groups of the previous, any conjugated organic compound that alternates in its structure double bonds, triple bonds or a combination of both with aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous, any non-conjugated organic compound that contains aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous or any combination thereof;
X₁, X₂ and X₃ are the same or different and are selected from any heteroatom,
Y is selected from a saturated or unsaturated alkyl group (C₂-C₁₂) or cycloalkyl (C₃-C₈), cycloalkenyl, aryl or heteroaryl,
R₁ and R₂ are the same or different bound to a primary, secondary or tertiary amino group and are selected from hydrogen, saturated or unsaturated alkyl (C₁-C₁₂), cycloalkyl (C₃-C₈), cycloalkenyl, aryl or heteroaryl,
n represents the number of branches and is a whole number selected from between 1 and 10.

3. Compound of general formula (I) or (II) according to any of the preceding claims, wherein they may be in the form of salt and electrostatically bound to trifluoroacetate, chloride anion, DNA, RNA, siRNA, miRNA, antagomir, any drug, antibody, probe for the diagnosis of diseases by imaging techniques or any combination thereof.

4. Compound of general formula (I) or (II) according to any of claims 1 to 3, wherein X₁, X₂ and X₃ are the same or different and are selected from nitrogen or oxygen.

5. Compound of general formula (I) or (II) according to any of claims 1 to 4, wherein Y is selected from:

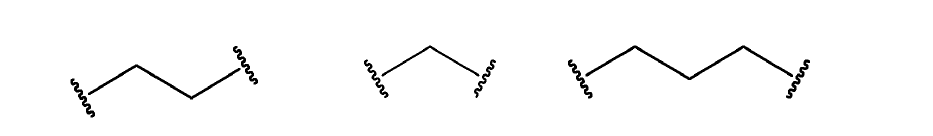

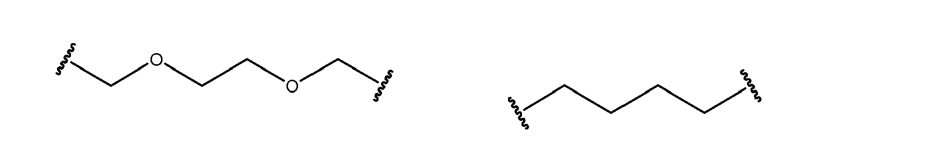

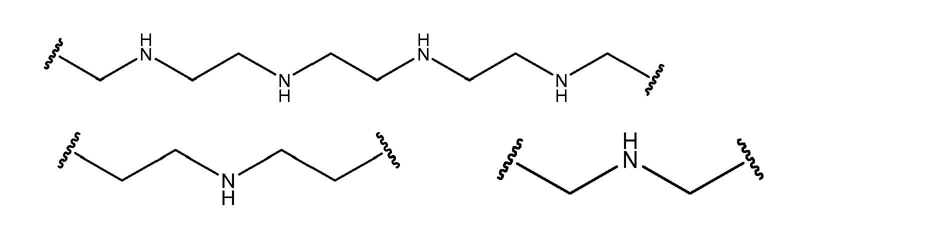

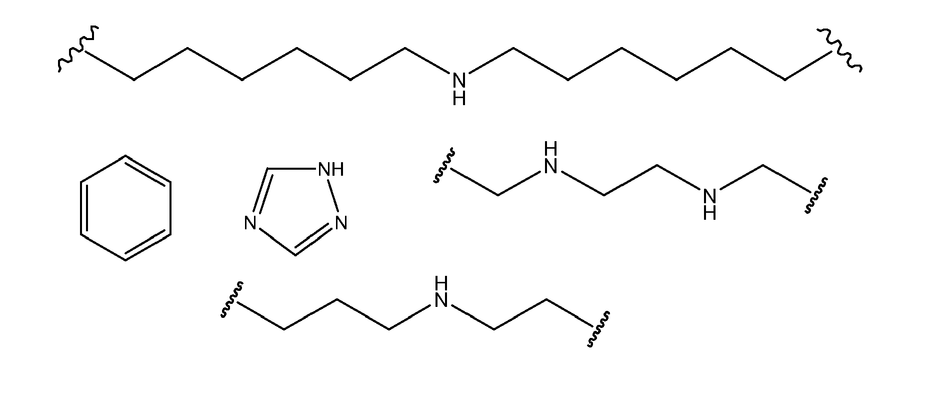

6. Compound of formula (I), or (II) according to any of claims 1 to 5, wherein R₁ to R₂ may be the same or different and are independently selected from H, any substituted or non-substituted amine, basic amino acids, derivatives of cholesterol, folic acid, lactic acid, dexamethasone, sugars, lysosomotropic agents, nitrogenated heterocycles, polyethylene glycol-derivative hydrophilic chains, any diacrylate, any basic amino acid and the following structures:

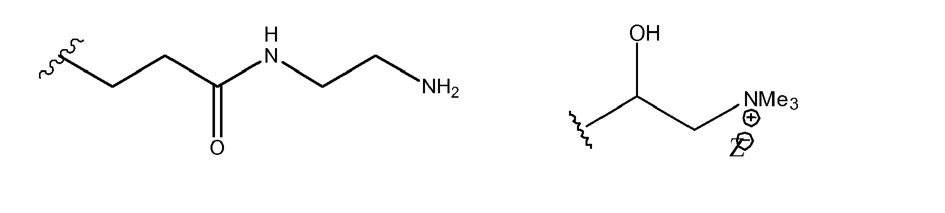

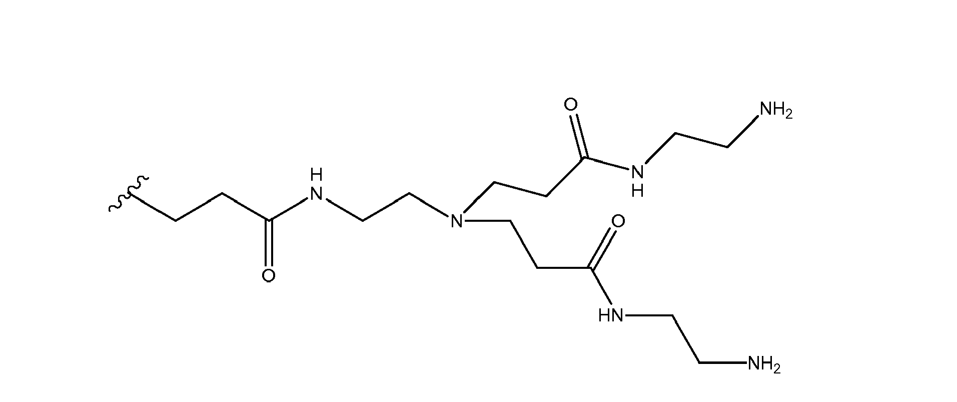

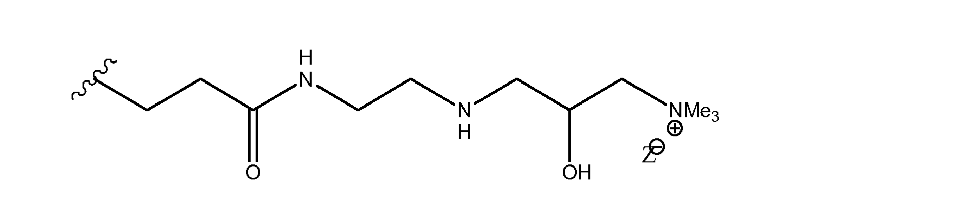

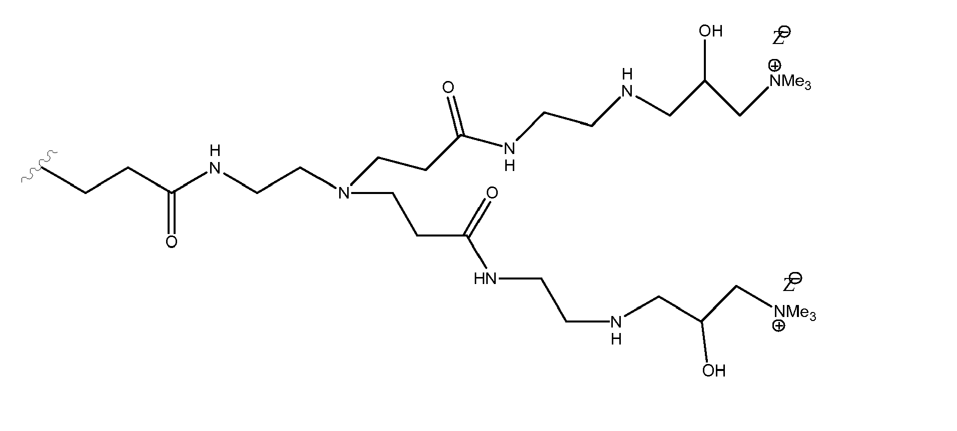

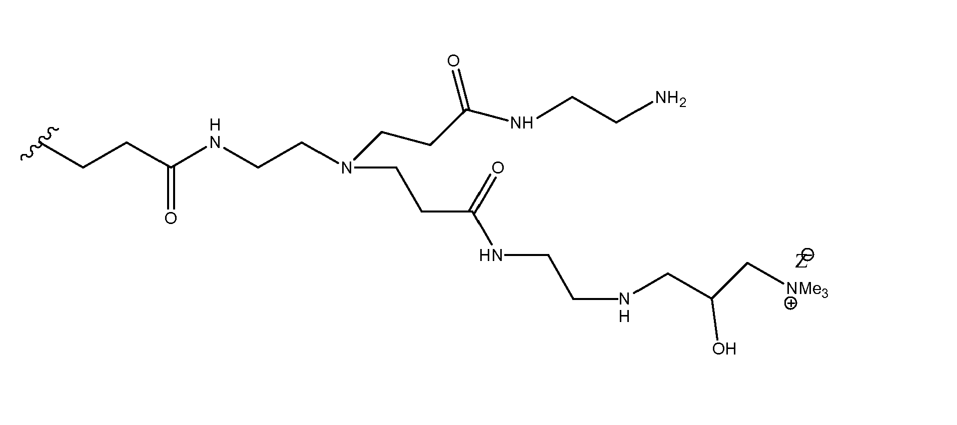

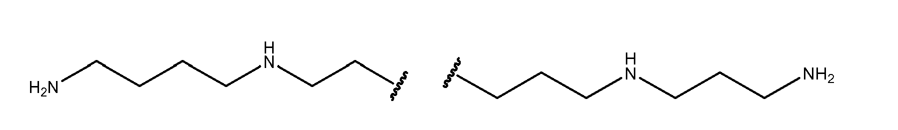

7. Compound of general formula (I), or (II) according to any of claims 1 to 6 selected from the list comprising:

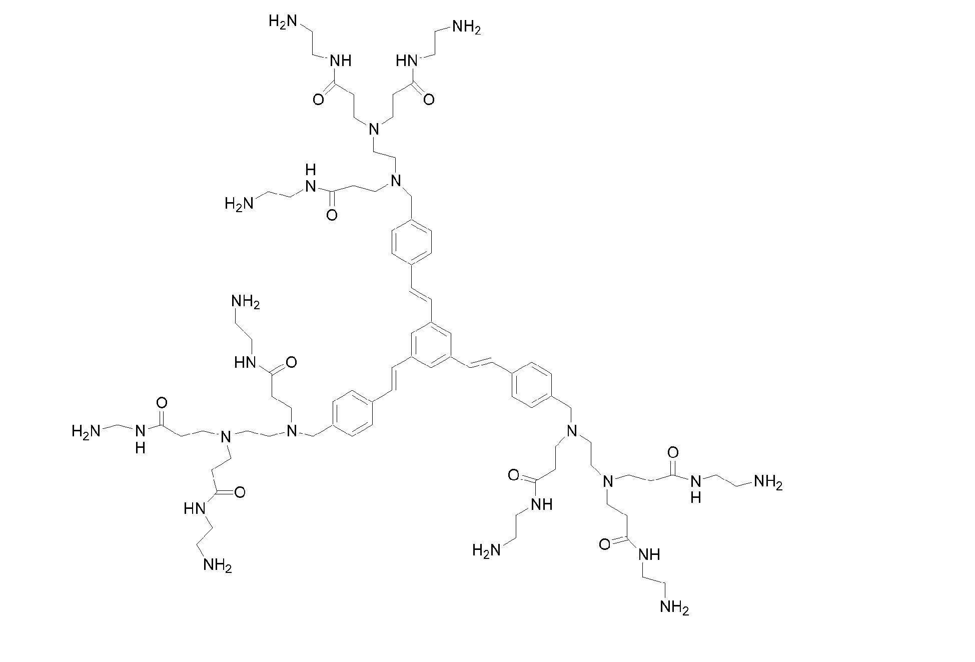

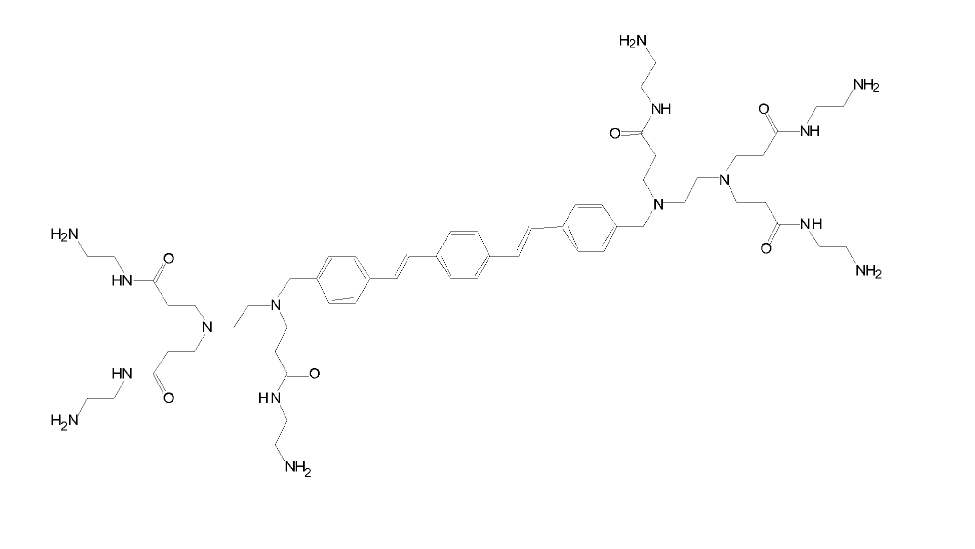

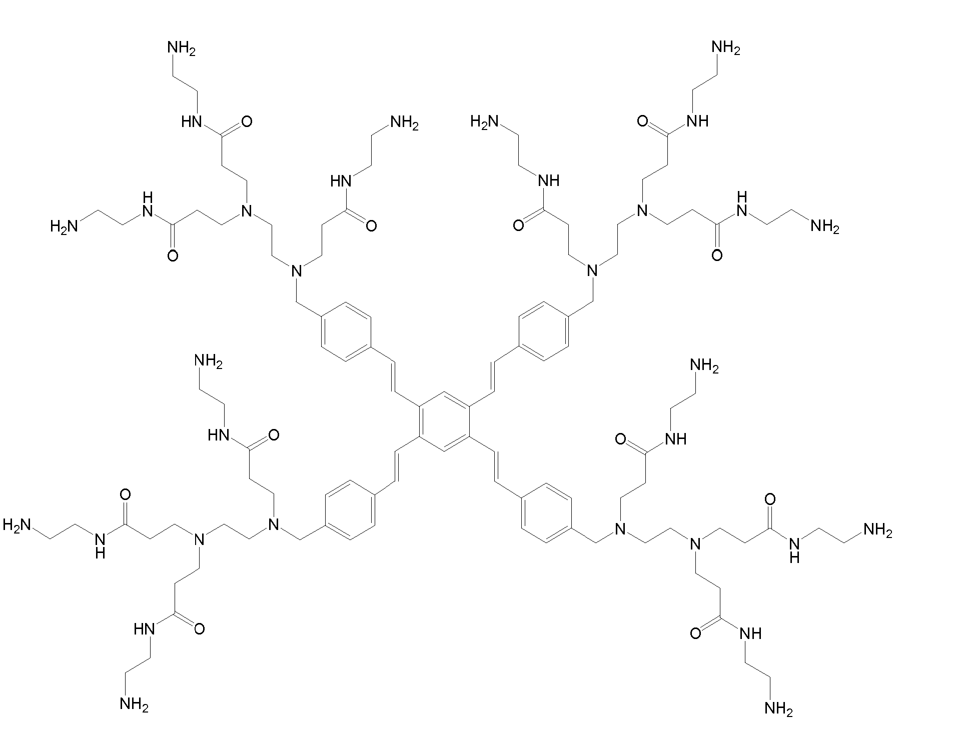

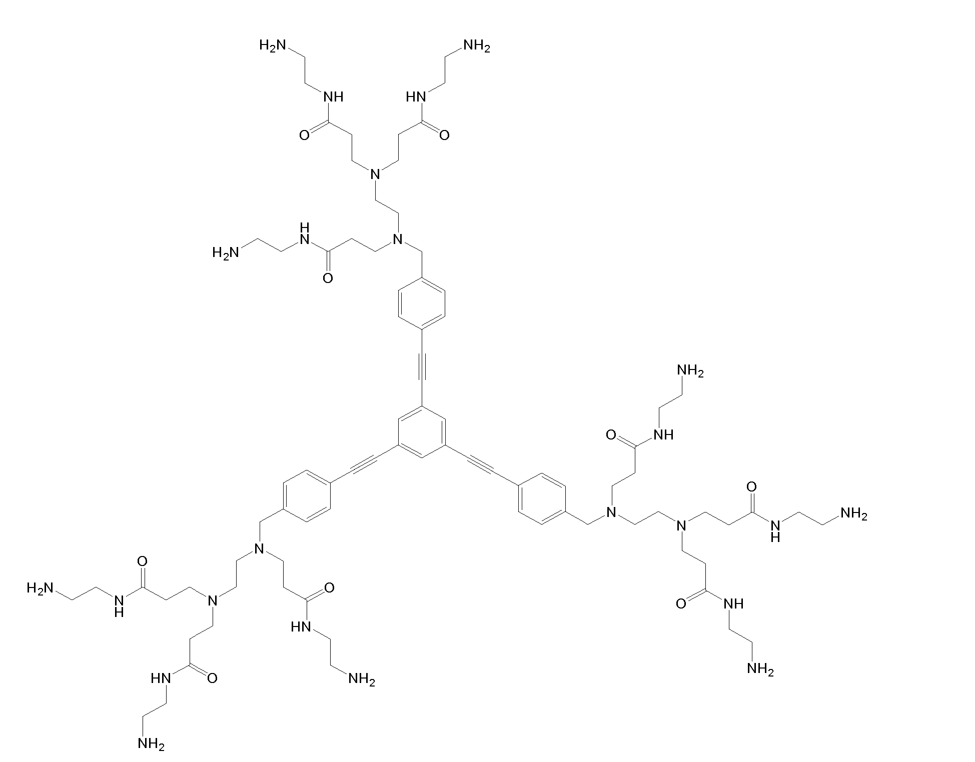

8. Pharmaceutical composition, **characterized in that** it comprises a compound of formula **(I)** according to any of claims 1, 3, 4 5 and 6 together with one or more pharmaceutically acceptable excipients.

9. Pharmaceutical composition **characterized in that** it comprises a compound of formula **(II)** according to any of claims 2, 3, 4 5 and 6, together with one or more pharmaceutically acceptable excipients.

10. Composition according to any of claims 8 and 9, **characterized in that** it further comprises one or more active principles.

11. Process of synthesis of the compounds of general formula (I), **characterized in that** it comprises the following stages: 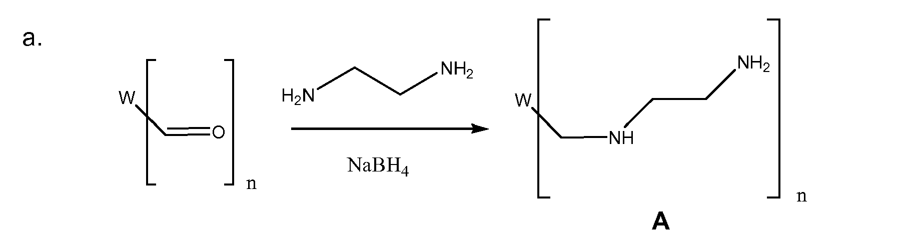 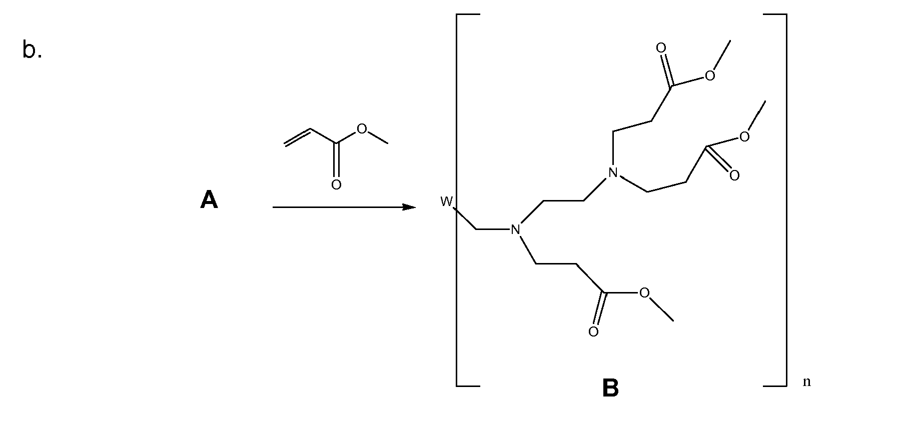 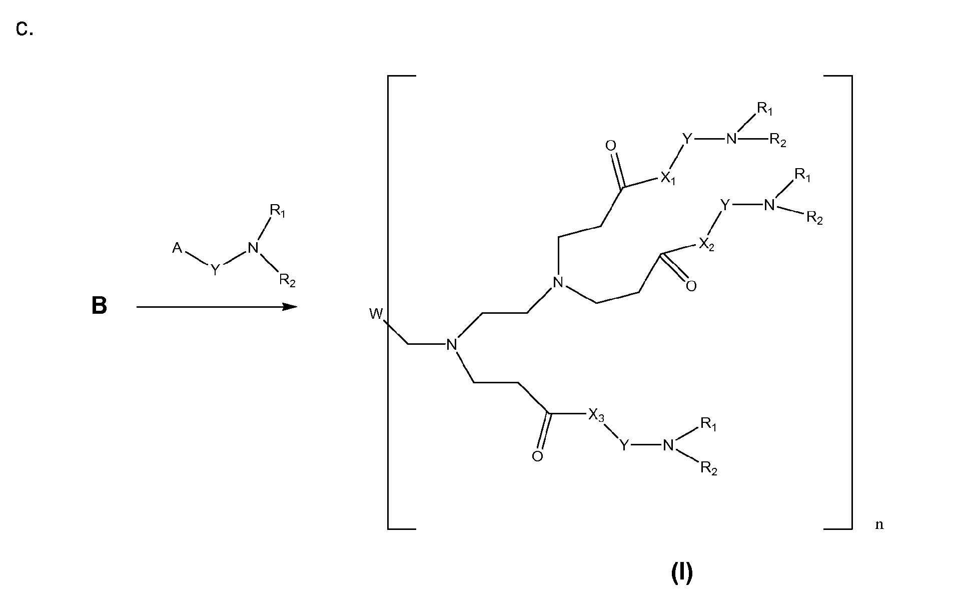 wherein:
W is selected from the group formed by any polymer or dendrimer derived from polyphenylenevinylene, polyphenyleneethylidene or hybrid of both, any conjugated organic compound that combines double bonds, triple bonds or a composition of both in its structure, any substitution with electron-donor or electro-attractor groups of the previous, any conjugated organic compound that alternates in its structure double bonds, triple bonds or a combination of both with aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous, any non-conjugated organic compound that contains aromatic rings of phenyl, naphthalene, phenanthrene, anthracene, pyrrole, furan, thiophene, pyridine, aromatic heterocyclic bases such as cytosine, uracil, adenine, thymine and guanine, or any substitution with electron-donor or electro-attractor groups of the previous or any combination thereof;
A is selected from X₁, X₂ and X₃
X₁, X₂ and X₃ are the same or different and are selected from any heteroatom,
Y is selected from a saturated or unsaturated alkyl group (C₂-C₁₂) or cycloalkyl (C₃-C₈), cycloalkenyl, aryl or heteroaryl,
R₁ and R₂ are the same or different bound to a primary, secondary or tertiary amino group and are selected from hydrogen, saturated or unsaturated alkyl (C₁-C₁₂), cycloalkyl (C₃-C₈), cycloalkenyl, aryl or heteroaryl, and
n represents the number of branches and is a whole number selected from between 1 and 10.

12. Process for the obtainment of a compound of general formula (II), wherein it starts from the compound of general formula (I) and it repeats all steps a-c according to claim 10.

13. Use of a compound of general formula (I) or (II) according to any of claims 1 to 7 for the preparation of a medicament.

14. Use of the compound of general formula (I) or (II) according to claim 13 for the preparation of a medicament for the prevention and/or treatment of pathologies such as nervous system diseases, any disease that presents the appearance of tumours, osteoporosis, diabetes, peritoneal dialysis and viral infections, including those caused by the human immunodeficiency syndrome virus (AIDS).

15. Use of the compound of general formula (I) or (II) according to any of claims 1 to 6 for the preparation of a siRNA transfection kit in primary cultures of nerve cells, glia, tumoral cells, fibroblasts, osteoblasts and primary cells.

16. Use of the compound of general formula (I) or (II) according to any of claims 1 to 6 in gene therapy as non-viral vector.

17. Use of the compound of general formula (I) or (II) according to any of claims 1 to 6 for the preparation of probes for the diagnosis of diseases by imaging techniques.

18. Use of the compound of general formula (I) or (II) according to any of claims 1 to 6, for the selective delivery of drugs, genetic material or cell line image probes
